# EUROPEAN PATENT SPECIFICATION

(11) **EP 3 952 901 B1**
(45) Date of publication and mention of the grant of the patent: **02.07.2025**
(21) Application number: 20716827.9
(22) Date of filing: 09.04.2020
(51) Int. Cl.: A61K 38/02, A61K 35/00

(54) **INTERLEUKIN-4-INDUCED GENE 1 (IL4I1) AS A BIOMARKER AND USES THEREOF**
INTERLEUKIN-4-INDUZIERTES GEN 1 (IL4I1) ALS BIOMARKER UND VERWENDUNGEN DAVON
GÈNE 1 INDUIT PAR L'INTERLEUKINE-4 (IL4I1) EN TANT QUE BIOMARQUEUR ET SES UTILISATIONS

(30) Priority: 10.04.2019 EP 19168304
(43) Date of publication of application: 16.02.2022
(73) Proprietor: Deutsches Krebsforschungszentrum Stiftung des öffentlichen Rechts, 69120 Heidelberg (DE)
(72) Inventor: OPITZ, Christiane, 69120 Heidelberg (DE); SADIK, Ahmed, 69120 Heidelberg (DE); SOMARRIBAS PATTERSON, Luis Felipe, 69120 Heidelberg (DE); MOHAPATRA, Soumya, 69120 Heidelberg (DE); PRENTZELL, Mirja Tamara, 69120 Heidelberg (DE); SECKER, Philipp, 69120 Heidelberg (DE); TRUMP, Saskia, 04105 Leipzig (DE)
(74) Representative: Kilger, Ute
(86) International application number: PCT/EP2020/060259
(87) International publication number: WO 2020/208190

(56) References cited:
- WO-A1-2010/066858
- WO-A1-2017/189353
- YINPU YUE ET AL: "IL4I1 Is a Novel Regulator of M2 Macrophage Polarization That Can Inhibit T Cell Activation via L-Tryptophan and Arginine Depletion and IL-10 Production", PLOS ONE, vol. 10, no. 11, 24 November 2015 (2015-11-24), pages e0142979, XP055383651, DOI: 10.1371/journal.pone.0142979

## Description

The present invention relates to a newly identified AHR-activating enzyme and uses thereof as a marker in diagnosis and therapy, for example for selecting patients for treatment with IL4I1 modulating interventions and monitoring of therapy response.

### Background of the invention

IL4I1 is an L-amino acid oxidase that catalyzes the oxidative deamination of L-amino acids to alpha-keto acids while producing hydrogen peroxide and ammonia (1). Initially discovered as an immediate-early IL4-inducible gene in B cells (2, 3), IL4I1 was later identified also in macrophages and dendritic cells (4). In addition, IL4I1 is expressed in human malignancies either in the neoplastic cells themselves, or in tumor-associated macrophages (5). IL4I1 inhibits T cell proliferation (4, 6), which has mainly been attributed to its H₂O₂ production. Moreover, II,4I1 has been implicated in Th17 cell (7) and regulatory T cell (8) differentiation, which is known to be modulated by AHR activation (9-11).

Currently, only very little is known about the therapeutic implications of IL4I1-associated conditions. This can be exemplified by the presence of only one patent publication, WO 2016/040488, which discloses methods of promoting myelin formation in central nervous system (CNS) tissue in a subject in need thereof, the method comprising administering to the subject a therapeutically effective amount of IL4I1 protein. WO 2017/189353 discloses the IL4I1 activity may be reduced with antagonists, including, antibodies, aptamers and proteins.

The aryl hydrocarbon receptor (AHR) is a ligand-activated transcription factor involved in the regulation of diverse processes such as embryogenesis, vasculogenesis, metabolism, immunity and cancer. In preclinical studies, AHR activation by tryptophan metabolites generated through indoleamine-2,3-dioxygenase (IDO1) and/or tryptophan-2,3-dioxygenase (TDO2) promoted tumor progression by enhancing the motility, anoikis resistance and clonogenic survival of the tumor cells as well as by suppressing anti-tumor immune responses (12). AHR target gene expression is context specific (13), and the introduction of new biomarkers of AHR activation that efficiently detect AHR activation across different cells/tissues and in response to diverse AHR ligands is required. Furthermore, the functional implications of IL4I1 modulation and AHR modulation share many common pathways and cross talk, entailing the importance of considering IL4I1 as a potential biomarker for conditions of AHR modulation and vice versa.

In a first aspect thereof, the present invention relates to a method for detecting a modulation of AHR in a cell or a subject, comprising detecting a change of the biological state of IL4I1 in said cell or a biological sample derived from said subject, wherein a change in said biological state of IL4I1 in said cell or sample, when compared to a control cell or sample, e.g. a sample derived from a healthy subject, a patient or patient group, indicates an IL4I1-related modulation of AHR in said cell or subject.

In a second aspect thereof, the present invention provides an *in vitro* method for screening for at least one potential modulator of the expression and/or biological activity of IL4I1, comprising contacting a sample comprising IL4I1 or a cell expressing IL4I1 with at least one candidate modulator compound, and detecting a modulation of said IL4I1, wherein said modulation identifies a potential modulator of the expression and/or biological activity of IL4I1.

Preferably, the method is a method for screening for at least one modulator of the biological state of IL4I1, comprising contacting at least one candidate modulator compound with a biological sample and detecting a change of said biological state of IL4I1 in a biological sample, wherein a change in the biological state of said IL4I1 in the presence of said at least one modulator compared to the absence of said at least one modulator identifies a modulator.

In a third aspect thereof, the present invention relates to a method for monitoring the modulation of the biological state of IL4I1 in response to at least one compound, comprising performing a method according to the present invention on a biological sample that was contacted with at least one compound, and wherein said biological sample is compared to a control sample that was not contacted with said compound.

In a sub-aspect of this method, the present invention relates to a method for monitoring the biological state of AHR in a cell, comprising providing at least one compound to said cell and detecting the change in the biological state, such as the expression and/or biological function of IL4I1 in said cell in response to said at least one compound, wherein a change in the biological state, such as in the expression or biological function in the presence of said at least one compound compared to the absence of said at least compound indicates an effect of said at least one compound on said biological state of AHR in a cell.

In another preferred aspect of the present invention, the invention then relates to a method for treating and/or preventing an AHR-related disease or condition in a cell, for example in a patient in need of said treatment, comprising performing a method according to the present invention, and providing a suitable treatment to said patient, wherein said treatment is based, at least in part, on the results of the method according to the present invention, such as providing a compound as identified or monitoring a treatment comprising the method(s) as described herein.

Another important aspect of the present invention relates to a diagnostic kit comprising materials for performing a method according to the present invention in one or separate containers, optionally together with auxiliary agents and/or instructions for performing said method. Another important aspect of the present invention then relates to the use of said diagnostic kit in a method according to the present invention.

Finally, the invention relates to the use of the biomarker IL4I1 for screening for modulators according to the present invention or for monitoring according to the present invention.

Generally preferred are the human variants of the biomarker IL4I1, or closely related species, like the ones from other primates or mammals.

Other aspects and advantages can be readily derived from reading the following description and the non-limiting examples.

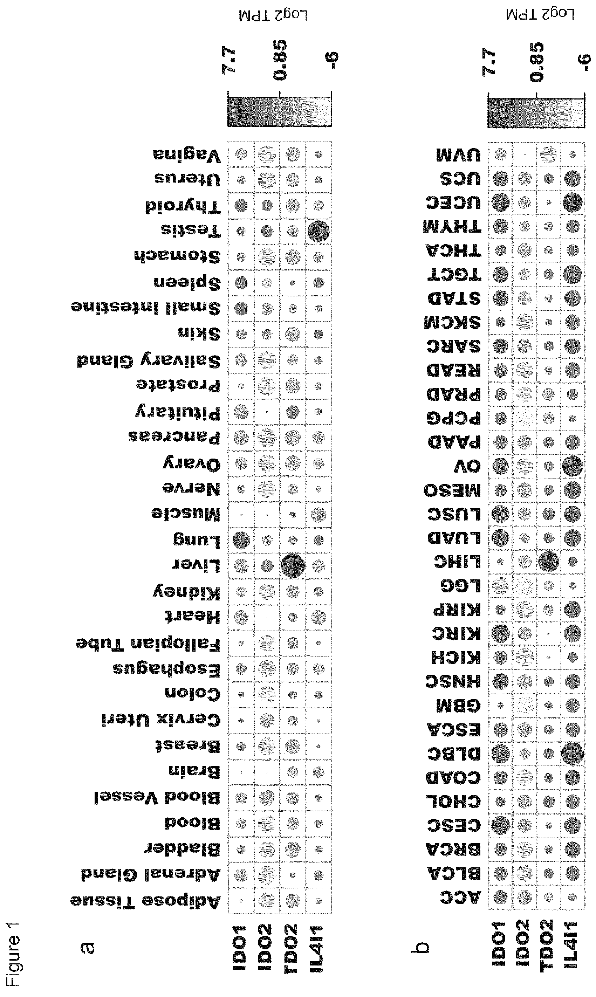

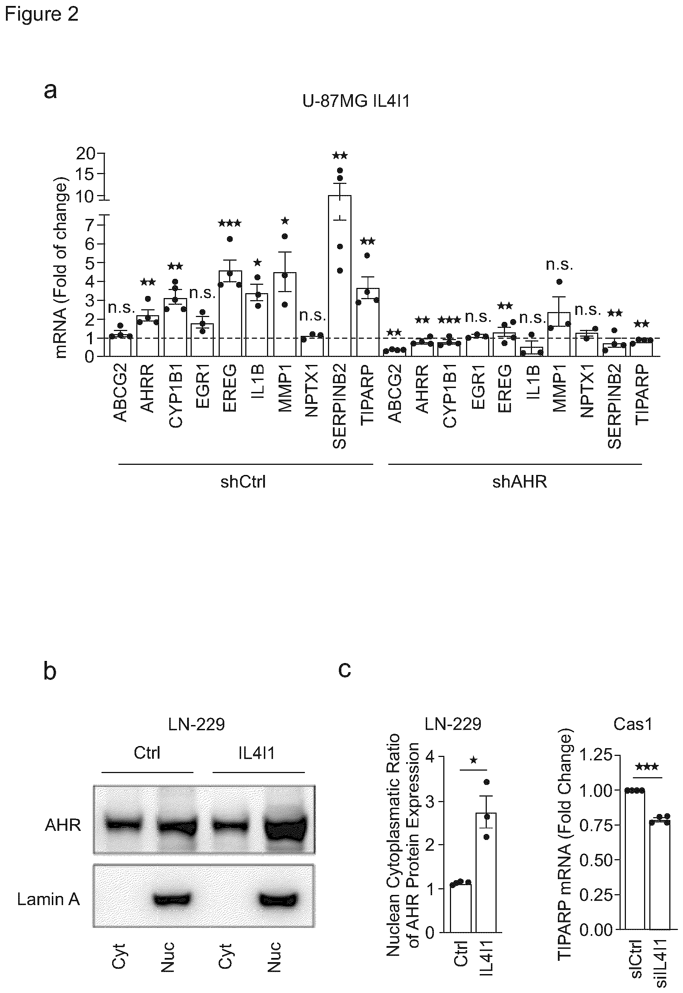

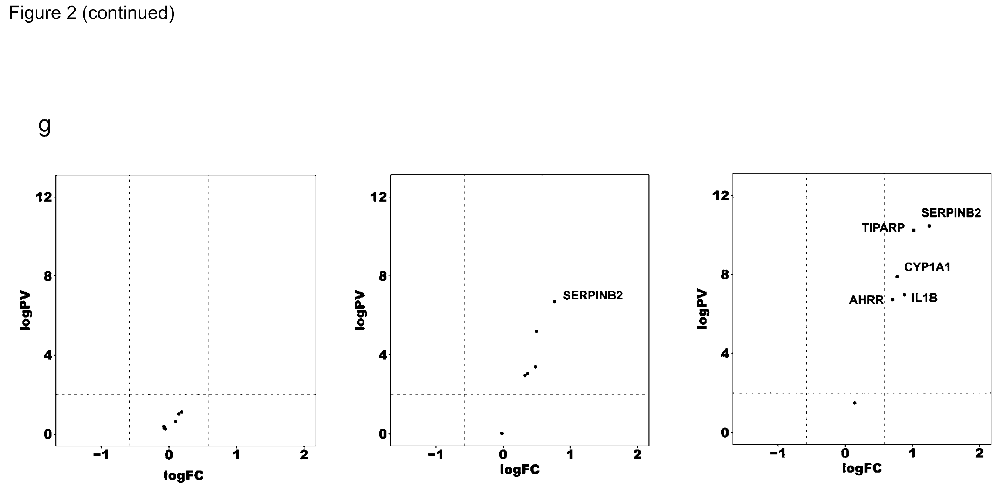

The present inventors - while investigating the role of tryptophan degrading enzymes in modulating AHR activity - discovered that the expression of IL4I1, a tryptophan-degrading enzyme expressed in human cancers (Fig. 1) and not yet implicated in AHR activation, correlated significantly with AHR target gene expression. Gene expression analyses (Fig. 2a) and AHR nuclear translocation (Fig. 2b) established IL4I1 to activate the AHR via production of tryptophan metabolites including kynurenic acid (Fig. 2g-i, Fig. 3-4).

As mentioned above, in experiments as performed in the context of the present invention, the new biomarker IL4I1 was identified as a new component upstream of the AHR. This enables an *in vitro* method for screening for at least one modulator of the biological state of IL4I1, comprising contacting at least one candidate modulator compound with a biological sample and detecting a change of said biological state of IL4I1 in a biological sample, wherein a change in the biological state of said IL4I1 in the presence of said at least one modulator compared to the absence of said at least one modulator identifies a modulator. A modulator can be an activator (inducer) or inhibitor of said biological state of IL4I1.

In one alternative, the method for screening for a potential modulator of the expression and/or biological activity of IL4I1 comprises contacting a sample comprising IL4I1 or a cell expressing IL4I1 with at least one candidate modulator compound and detecting a binding of said modulator to said IL4I1, wherein said binding identifies a potential modulator of the expression and/or biological activity of IL4I1. This method preferably further comprises the step of detecting the expression and/or biological activity of IL4I1 in said cell or the biological activity of IL4I1 in said sample, wherein a change in the expression or biological activity of IL4I1 in the presence of said at least one compound compared to the absence of said at least one compound identifies a modulator.

Preferred is such a method according to the present invention, wherein said modulator is selected from an inhibitor or an inducer of said expression or biological activity.

Another important aspect of the present invention then relates to a method for diagnosing an AHR-related disease or condition in a cell and/or a subject, comprising detecting a change of the biological state of IL4I1 in a biological sample derived from said cell and/or subject, wherein a change in said biological state of IL4I1 in said sample, when compared to a control sample, indicates an AHR-related physiological or pathological condition in said cell and/or subject.

In one aspect, said method comprises detecting the expression or biological function of IL4I1 in a cell/tissue/biological fluid, wherein a change in the expression or biological function in said compartments, in particular expression or activation, when compared to a healthy or other suitable control sample, indicates an AHR-related disease or condition. Such a change can be at least about 20%, at least about 30%, at least about 40%, at least about 50%, at least about 60%, at least about 70%, at least about 80% or at least about 90% or more of up or down regulation of expression or biological function, when compared with a suitable control, such as the value in a healthy cell or a sample derived from a healthy person or group of individuals, or when compared to an internal standard, like a housekeeping gene. In the context of the present invention, the term "about" shall mean +/- 10% of the given value, unless indicated otherwise.

In the context of the present invention, it was found that said modulation of AHR in a cell or subject as detected through detecting a change of the biological state of IL4I1 is indicative for an AHR-related physiological or pathological condition in said cell or subject. Preferably, the AHR-associated physiological or pathological condition to be stratified and/or diagnosed is selected from intoxication, cancer, autoimmune disorders, degeneration, inflammation, infection, metabolic diseases and conditions, angiogenesis, drug metabolism, hematopoiesis, lipid metabolism, cell motility, immune modulation, and stress conditions, for example, biological, mechanical and environmental stresses.

Preferred is a method according to the present invention, wherein said biological state as detected is selected from mutations, nucleic acid methylation, copy numbers, expression, amount of protein, protein modifications, cellular localization, metabolites, in particular metabolites as produced by IL4I1, such as, for example, tryptophan degradation products, tryptophan metabolites including kynurenic acid, and the biological activity of IL4I1.

In a preferred embodiment, in the method according to the present invention said biological state of IL4I1 is detected indirectly through a change of abundance or biological activity of at least one metabolite biomarker according to table 1 as herein below, wherein a change in said expression or biological activity of said at least one biomarker when compared to a control sample indicates a change of the biological state of IL4I1 in said sample.

In the method according to the present invention, said biological sample can be selected from a suitable sample comprising biological fluids, human cells, tissues, whole blood, cell lines, cellular supernatants, primary cells, IPSCs, hybridomas, recombinant cells, stem cells, and cancer cells, bone cells, cartilage cells, nerve cells, glial cells, epithelial cells, skin cells, scalp cells, lung cells, mucosal cells, muscle cells, skeletal muscles cells, striated muscle cells, smooth muscle cells, heart cells, secretory cells, adipose cells, blood cells, erythrocytes, basophils, eosinophils, monocytes, lymphocytes, T-cells, B-cells, neutrophils, NK cells, regulatory T-cells, dendritic cells, Th17 cells, Th1 cells, Th2 cells, myeloid cells, macrophages, monocyte derived stromal cells, bone marrow cells, spleen cells, thymus cells, pancreatic cells, oocytes, sperm, kidney cells, fibroblasts, intestinal cells, cells of the female or male reproductive tracts, prostate cells, bladder cells, eye cells, corneal cells, retinal cells, sensory cells, keratinocytes, hepatic cells, brain cells, kidney cells, and colon cells, and the transformed counterparts of said cells or tissues.

Preferred is a method according to the present invention, wherein said subject is selected from a mammalian subject, in particular a human subject, in particular a human patient suffering from an AHR-related physiological or pathological condition. Said control sample can be selected from a sample as described above.

Another aspect of the invention then relates to a method for monitoring the modulation of the biological state of IL4I1 in response to at least one compound, comprising performing a method according to the present invention on a biological sample that was contacted with an amount of said at least one compound, and wherein said biological sample is compared to a control sample that was not contacted with said amount of said compound.

Preferred is such a method for monitoring an AHR-related disease or condition in a cell, comprising providing at least one compound to said cell and detecting the change in the expression or biological function of IL4I1 in said cell in response to said at least one compound, wherein a change in the expression or biological function in the presence of said at least one compound compared to the absence of said at least compound indicates an effect of said at least one compound on said IL4I1-related disease or condition.

In the context of the invention, the AHR-related disease or condition can be selected from at least one of intoxication, cancer, autoimmune disorders, degeneration, inflammation, infection, metabolic diseases and conditions, angiogenesis, drug metabolism, hematopoiesis, lipid metabolism, cell motility, senescence, immune modulation, stress conditions, for example, biological, mechanical and environmental stresses, and AHR modulation.

In some embodiments, the condition is cancer. In some embodiments, the cancer is selected from Adrenocortical carcinoma(ACC), Bladder Urothelial Carcinoma (BLCA), Breast invasive carcinoma (BRCA), Cervical squamous cell carcinoma and endocervical adenocarcinoma (CESC), Cholangiocarcinoma (CHOL), Colon adenocarcinoma (COAD), Lymphoid Neoplasm Diffuse Large B-cell Lymphoma (DLBC), Esophageal carcinoma (ESCA), Glioblastoma multiforme (GBM), Head and Neck squamous cell carcinoma (HNSC), Kidney Chromophobe (KICH), Kidney renal clear cell carcinoma (KIRC), Kidney renal papillary cell carcinoma (KIRP), Brain Lower Grade Glioma (LGG), Liver hepatocellular carcinoma (LIHC), Lung adenocarcinoma (LUAD), Lung squamous cell carcinoma (LUSC), Mesothelioma (MESO), Ovarian serous cystadenocarcinoma (OV), Pancreatic adenocarcinoma (PAAD), Pheochromocytoma and Paraganglioma (PCPG), Prostate adenocarcinoma (PRAD), Rectum adenocarcinoma (READ), Sarcoma (SARC), Skin Cutaneous Melanoma (SKCM), Stomach adenocarcinoma (STAD), Testicular Germ Cell Tumors (TGCT), Thyroid carcinoma (THCA), Thymoma (THYM), Uterine Corpus Endometrial Carcinoma (UCEC), Uterine Carcinosarcoma (UCS), and Uveal Melanoma (UVM).

Methods according to the present invention are provided to, in one aspect, seek for modulators and elucidate effects thereof on the biomarker IL4I1 as identified. Examples of such compounds to be identified can be selected from a small molecule, a peptide, and a library of said compounds. Said compound as identified (screened) is selected from small chemical molecules, peptides, antibodies, and short interfering RNAs. Preferably, said compound can be selected from a proteinaceous domain, a small molecule, a peptide, an environmental substance, probiotic, toxin, aerosol, medicine, nutrient, galenic composition, plant extract, volatile compound, homeopathic substance, incense, pharmaceutical drug, vaccine, a compound or compound mixture derived from organisms, for example animals, plants, fungi, bacteria, archaea, a chemical compound, a compound used in food or cosmetic industry, and a library of said compounds.

Identification and screening using the biomarkers as disclosed herein can be done using respective methods known in the art, preferably using recombinantly produced proteins of the biomarkers, and/or recombinant cell models. For this, the biomarkers can be labeled, e.g. using chemical dyes or fluorescent markers. Additionally, enzymes can be used, optionally in the form of fusions with the biomarker to be screened. Preferably, said method is also amenable to automatization, and said screening is preferably assessed in an automated and/or high-throughput format.

Another aspect then relates to the use of at least one biomarker of IL4I1 for screening for modulators according to the present invention or for monitoring according to the present invention or for testing the biological safety according to the present invention or for a diagnosis according to the present invention.

In another preferred aspect of the present invention, the invention then relates to a method of treating and/or preventing an AHR-related disease or condition in a cell in a patient in need of said treatment, comprising performing a method according to the present invention, and providing a suitable treatment to said patient, wherein said treatment is based, at least in part, on the results of the method according to the present invention, such as providing a compound as identified or monitoring a treatment.

In the context of the present invention, any biological sample comprising the marker protein IL4I1 (or functionally relevant parts thereof), or a sample comprising cells, comprising the marker protein IL4I1 (or functionally relevant parts thereof), e.g. obtained from a cancer patient, or a sample comprising at least one of the metabolites produced downstream of IL4I1, for example as shown in Table 1, can be used, as long as it contains (or is presumed to contain) at least one of the biomarker(s) to be used in the analysis and/or screen. Preferably, the biological sample is selected from a sample comprising biological fluids comprising biomarkers, cells, a suitable sample comprising biological fluids, human cells, tissues, whole blood, cell lines, cellular supernatants, primary cells, IPSCs, hybridomas, recombinant cells, stem cells, and cancer cells, bone cells, cartilage cells, nerve cells, glial cells, epithelial cells, skin cells, scalp cells, lung cells, mucosal cells, muscle cells, skeletal muscles cells, striated muscle cells, smooth muscle cells, heart cells, secretory cells, adipose cells, blood cells, erythrocytes, basophils, eosinophils, monocytes, lymphocytes, T-cells, B-cells, neutrophils, NK cells, regulatory T-cells, dendritic cells, Th17 cells, Th1 cells, Th2 cells, myeloid cells, macrophages, monocyte derived stromal cells, bone marrow cells, spleen cells, thymus cells, pancreatic cells, oocytes, sperm, kidney cells, fibroblasts, intestinal cells, cells of the female or male reproductive tracts, prostate cells, bladder cells, eye cells, corneal cells, retinal cells, sensory cells, keratinocytes, hepatic cells, brain cells, kidney cells, and colon cells, and the transformed counterparts of said cells or tissues. The sample can also be selected from tumor tissue (tumor or metastases), biopsies, whole blood, peripheral blood, or fractions thereof, serum, buffy coat, lymphatic fluid, urine, bone marrow, heparinized whole blood, and frozen samples thereof, such as frozen heparinized whole blood. The cells to be used in the methods according to the present invention can be recombinant or non-recombinant, and express cell-foreign proteins, depending on the desired purpose and circumstances. Totipotent human embryonic stem cells may be excluded, if necessary. The sample can also be a combined sample from a group of subjects, for example, a patient group.

Another aspect of the present invention then relates to a method for producing a pharmaceutical preparation, wherein said compound/modulator as identified (screened) is further formulated into a pharmaceutical preparation by admixing said (at least one) compound as identified (screened) with a pharmaceutically acceptable carrier. Pharmaceutical preparations can be preferably present in the form of injectables, tablets, capsules, syrups, elixirs, ointments, creams, patches, implants, aerosols, sprays and suppositories (rectal, vaginal and urethral). Another aspect of the present invention then relates to a pharmaceutical preparation as prepared according to the invention.

In another aspect of the present invention, the invention then relates to a diagnostic kit comprising materials for performing a method according to the present invention as herein in one or separate containers, optionally together with auxiliary agents and/or instructions for performing said method according to the present invention.

"Treatment" shall mean a reduction and/or amelioration of the symptoms of the disease. An effective treatment achieves, for example, a shrinking of the mass of a tumor and the number of cancer cells. A treatment can also avoid (prevent) and reduce the spread of the cancer, such as, for example, affect metastases and/or the formation thereof. A treatment may be a naive treatment (before any other treatment of a disease had started), or a treatment after the first round of treatment (e.g. after surgery or after a relapse). The treatment can also be a combined treatment, involving, for example, chemotherapy, surgery, and/or radiation treatment.

In the methods of the present invention, in general the biomarkers can be detected and/or determined using any suitable assay. Detection is usually directed at the qualitative information ("marker yes-no"), whereas determining involves analysis of the quantity of a marker (e.g. expression level and/or activity). Detection is also directed at identifying for example mutations that cause altered functions of individual markers. The choice of the assay(s) depends on the parameter of the marker to be determined and/or the detection process. Thus, the determining and/or detecting can preferably comprise a method selected from subtractive hybridization, microarray analysis, DNA sequencing, RNA sequencing, qPCR, ELISA, IP, PLA, BiFC, HPLC, WB, enzymatic activity tests, fluorescence detection, cell viability assays, for example an MTT assay, phosphoreceptor tyrosine kinase assays, phospho-MAPK arrays and proliferation assays, for example the BrdU assay, proteomics, cytokine arrays, and mass spectrometry.

Preferably, said method is also amenable to automation, and said activity and/or expression is preferably assessed in an automated and/or high-throughput format. Usually, this involves the use of chips and respective machinery, such as robots.

Another aspect of the instant disclosure is directed to methods for determining the AHR activation state of a biological sample. In some embodiments, the biological sample is taken from a subject. In some embodiments, a biological state is determined/measured for Interleukin 4-Induced gene 1 (IL4I1).

In some embodiments, the method for determining AHR activation signature for a condition comprises: (a) obtaining a biological sample from a subject ; (b) determining, in the biological sample, a biological state of IL4I1; (c) determining, in a control cell, the biological state of IL4I1; (d) comparing he biological state in step (b) to the biological state in step (c); and (e) determining the AHR activation state of biological sample based on the comparing.

In some embodiments, the biological state detected at step (b) is RNA expression. In some embodiments, the detecting a biological state comprises measuring levels of the biological state. In some embodiments, RNA expression of a biomarker is detected by methods known in the art including, but not limited to, qPCR, RT-qPCR, RNA-Seq, and in-situ hybridization.

In some embodiments, the method further comprises treating the subject with an AHR signaling modulator (also "AHR modulator"). In some embodiments, the AHR signaling modulator is administered every day, every other day, twice a week, once a week once a month or twice a month. In some embodiments, the AHR signaling modulator is administered together with other drugs as part of a combination therapy.

An "AHR signaling modulator" or an "AHR modulator" as used herein, refers to a modulator which affects AHR signaling in a cell. In some embodiments, an AHR signaling modulator exhibits direct effects on AHR signaling. In some embodiments, the direct effect on AHR is mediated through direct binding to AHR. In some embodiments, a direct modulator exhibits full or partial agonistic and/or antagonistic effects on AHR. In some embodiments, an AHR modulator is an indirect modulator.

In some embodiments, an AHR signaling modulator is a small molecule compound. The term "small molecule compound" herein refers to small organic chemical compound, generally having a molecular weight of less than 2000 daltons, 1500 daltons, 1000 daltons, 800 daltons, or 600 daltons.

In some embodiments, an AHR modulator comprises a 2-phenylpyrimidine-4-carboxamide compound, a sulphur substituted 3-oxo-2,3-dihydropyridazine-4-carboxamide compound, a 3-oxo-6-heteroaryl-2-phenyl-2,3-dihydropyridazine-4-carboxamide compound, a 2-hetarylpyrimidine-4-carboxamide compound, a 3-oxo-2,6-diphenyl-2,3-dihydropyridazine-4-carboxamide compound, a 2-heteroaryl-3-oxo-2,3-dihydro-4-carboxamide compound, PDM 2, 1,3-dichloro-5-[(1E)-2-(4-methoxyphenyl)ethenyl]-benzene, α-Naphthoflavone, 6, 2',4'-Trimethoxyflavone, CH223191, a tetrahydropyridopyrimidine derivative, StemRegenin-1, CH223191, GNF351, CB7993113 HP163 , PX-A590, PX-A548, PX-A275, PX-A758, PX-A446, PX-A24590, PX-A25548, PX-A25275, PX-A25758, PX-A26446, an Indole AHR inhibitor, and an oxazole-containing (OxC) compound.

In some embodiments, a direct AHR modulator comprises:
(a) Drugs: e.g. Omeprazole, Sulindac, Leflunomide, Tranilast, Laquinimod, Flutamide, Nimodipine, Mexiletine, 4-Hydroxy-Tamoxifen, Vemurafenib etc.
(b) Synthethic compounds: e.g. 10-Chloro-7H-benzimidazo[2,1-a]benz[de]isoquinolin-7-one (10-Cl-BBQ), Pifithrin-α hydrobromide,
(c) Natural compounds: e.g., kynurenine, kynurenic acid, cinnabarinic acid, ITE, FICZ, indoles including indole-3-carbinol, indole-3-pyruvate, indole-aldehyde, microbial metabolites, dietary components, quercetin, resveratrol, curcurmin, or
(d) Toxic compounds: e.g. TCDD, cigarette smoke, 3-methylcholantrene, benzo(a)pyrene, 2,3,7,8-tetrachlorodibenzofuran, fuel emissions, halogenated and nonhalogenated aromatic hydrocarbon, pesticides.

In some embodiments, indirect AHR modulators affect AHR activation through modulation of the levels of AHR agonists or antagonists.

In some embodiments, the modulation of the levels of AHR agonists or antagonists is mediated through one or more of the following:
(a) regulation of enzymes modifying AHR ligands e.g. the cytochrome p450 enzymes by e.g. cytochrome p450 enzyme inhibitors including 3'methoxy-4'nitroflavone (MNF), alpha-naphthoflavone (a-NF), fluoranthene (FL), phenanthrene (Phe), pyrene (PY) etc.
(b) regulation of enzymes producing AHR ligands including direct and indirect inhibitors/activators/inducers of tryptophan-catabolizing enzymes e.g. IDO1 pathway modulators (indoximod, NLG802), IDO1 inhibitors (1-methyl-L-tryptophan, Epacadostat, PX-D26116, navoximod, PF-06840003, NLG-919A, BMS-986205, INCB024360A, KHK2455, LY3381916, MK-7162, TDO2 inhibitors (680C91, LM10, 4-(4-fluoropyrazol-1-yl)-1,2-oxazol-5-amine, fused imidazo-indoles, indazoles), dual IDO/TDO inhibitors (HTI-1090/ SHR9146, DN1406131, RG70099, EPL-1410), immunotherapy incuding immune checkpoint inhibition, vaccination, and cellular therapies, chemotherapy, immune stimulants, radiotherapy, exposure to UV light, and targeted therapies such as e.g. imatinib etc.

In some embodiments, indirect AHR modulators affect AHR activation through modulation of the expression of the AHR including e.g. HSP 90 inhibitors such as 17-allylamino-demethoxygeldanamycin (17-AAG), celastrol.

In some embodiments, indirect AHR modulators affect AHR activation by affecting binding partners/co-factors modulating the effects of AHR including e.g. estrogen receptor alpha (ESR1).

Examples of AHR modulators are listed in US9175266, US2019/225683, WO2019101647A1, WO2019101642A1, WO2019101643A1, WO2019101641A1, WO2018146010A1, AU2019280023A1, WO2020039093A1, WO2020021024A1, WO2019206800A1, WO2019185870A1, WO2019115586A1, EP3535259A1, WO2020043880A1 and EP3464248A1, all of which are incorporated by reference in their entirety.

In some embodiments, an effective amount of a AHR signaling modulator is about 0.01 mg/kg to 100 mg/kg. In other embodiments, the effective amount of an AHR signaling modulator is about 0.01 mg/kg, 0.05 mg/kg, 0.1 mg/kg, 0.2 mg/kg, 0.5 mg/kg, 1 mg/kg, 5 mg/kg, 8 mg/kg, 10 mg/kg, 15 mg/kg, 20 mg/kg, 30 mg/kg, 40 mg/kg, 50 mg/kg, 60 mg/kg, 70 mg/kg, 80 mg/kg, 90 mg/kg, 100 mg/kg, 150 mg/kg, 175 mg/kg or 200 mg/kg of AHR signaling modulator.

Another aspect of the disclosure relates to a method of treating and/or preventing an AHR-related disease or condition in a cell in a patient in need of said treatment, comprising performing a method according to the present invention, and providing a suitable treatment to said patient, wherein said treatment is based, at least in part, on the results of the method according to the present invention, such as providing a compound as identified or monitoring a treatment comprising the method(s) as described herein.

Another aspect of the present disclosure relates to a diagnostic kit comprising materials for performing a method according to the present invention in one or separate containers, optionally together with auxiliary agents and/or instructions for performing said method.

Another aspect of the instant disclosure is directed to screening for or identifying compounds which modulate AHR activity. Another aspect of the instant disclosure is directed to methods for determining the effects of a compound on AHR activation status of a cell.

In some embodiments, a cell is treated with a candidate compound, and in the cell, a biological state of IL4I1 is determined/measured. In some embodiments, the biological state of IL4I1 in the biological sample is compared to the biological state of IL4I1 in a control sample. In some embodiments, the biological state is IL4I1 RNA expression.

In some embodiments, the candidate compound is categorized as an inhibitor of AHR signaling when the biological state of IL4I1 from the sample treated with a candidate compound is less than the biological state of IL4I1 from a control sample, and the candidate compound is categorized an activator of AHR signaling when the biological state the biological state of IL4I1 from the sample treated with a candidate compound is more than the biological state of IL4I1 from a control sample.

In some embodiments, a candidate compound is characterized as an AHR activator when it leads to at least 1.5 absolute fold upregulation in the biological state of IL4I1. In some embodiments, a candidate compound is characterized as an AHR activator when it leads to at least 2 absolute fold, at least 2.5 absolute fold, at least 3 absolute fold, at least 3.5 absolute fold, at least 4 absolute fold, at least 4.5 absolute fold, or at least 5 absolute fold upregulation in the biological state of IL4I1.

In some embodiments, a candidate compound is characterized as an AHR inhibitor when it leads to at least 0.67 absolute fold down-regulation in the biological state. In some embodiments, a candidate compound is characterized as an AHR inhibitor when it leads to at least 1 absolute fold, 2 absolute fold, at least 2.5 absolute fold, at least 3 absolute fold, at least 3.5 absolute fold, at least 4 absolute fold, at least 4.5 absolute fold, or at least 5 absolute fold down-regulation in the biological state.

The phrase "fold change" refers to the ratio between the value of a specific biomarker in two different conditions. In some embodiments, one of the two conditions could be a control. The phrase "absolute fold change" (which includes "absolute fold upregulation" and "absolute fold downregulation") is used herein in the case of comparing the log transformed value of a specific biomarker between two conditions. Absolute fold change is calculated by raising the exponent of the logarithm to the fold change value and then reporting the modulus of the number.

Various aspects of the present disclosure may be embodied as a program, software, or computer instructions embodied or stored in a computer or machine usable or readable medium, or a group of media which causes the computer or machine to perform the steps of the method when executed on the computer, processor, and/or machine. A program storage device readable by a machine, e.g., a computer readable medium, tangibly embodying a program of instructions executable by the machine to perform various functionalities and methods described in the present disclosure is also provided.

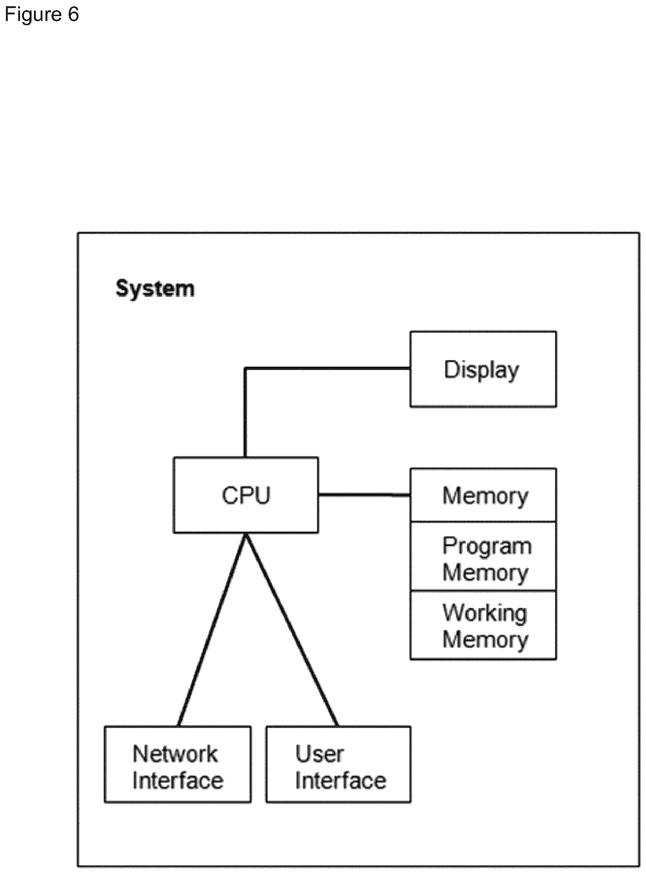

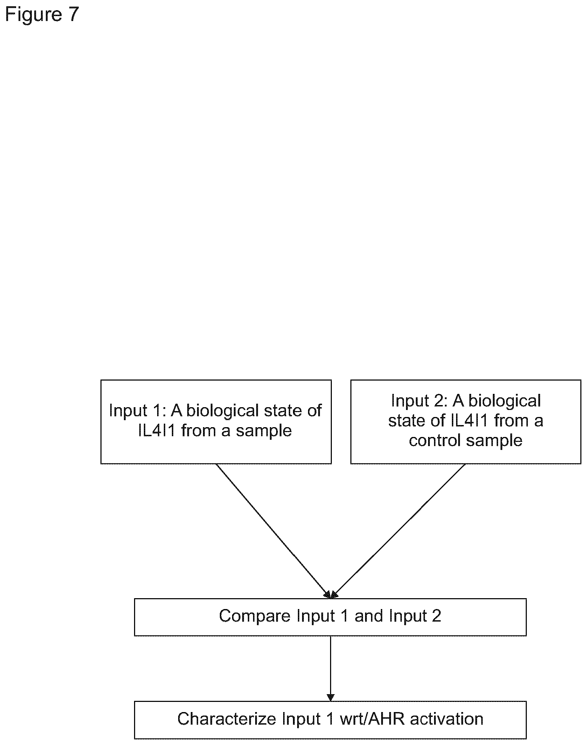

In some embodiments, the present disclosure includes a system comprising a CPU, a display, a network interface, a user interface, a memory, a program memory and a working memory (Figure 6), where the system is programmed to execute a program, software, or computer instructions directed to methods or processes of the instant disclosure. An exemplary embodiment is shown in Figure 7.

The term "processor" may include a single core processor, a multi-core processor, multiple processors located in a single device, or multiple processors in wired or wireless communication with each other and distributed over a network of devices, the Internet, or the cloud. Accordingly, as used herein, functions, features or instructions performed or configured to be performed by a "processor", may include the performance of the functions, features or instructions by a single core processor, may include performance of the functions, features or instructions collectively or collaboratively by multiple cores of a multi-core processor, or may include performance of the functions, features or instructions collectively or collaboratively by multiple processors, where each processor or core is not required to perform every function, feature or instruction individually. The processor may be a CPU (central processing unit). The processor may comprise other types of processors such as a GPU (graphical processing unit). In other aspects of the disclosure, instead of or in addition to a CPU executing instructions that are programmed in the program memory, the processor may be an ASIC (application-specific integrated circuit), analog circuit or other functional logic, such as a FPGA (field-programmable gate array), PAL (Phase Alternating Line) or PLA (programmable logic array).

The CPU is configured to execute programs (also described herein as modules or instructions) stored in a program memory to perform the functionality described herein. The memory may be, but not limited to, RAM (random access memory), ROM (read-only memory) and persistent storage. The memory is any piece of hardware that is capable of storing information, such as, for example without limitation, data, programs, instructions, program code, and/or other suitable information, either on a temporary basis and/or a permanent basis.

In some embodiments, the disclosure is directed to a processor is programmed to perform:
(a) comparing a biological state of Interleukin 4-Induced gene 1 (IL4I1) from a sample to the biological state of IL4I1 from a control sample; and
(e) determining the AHR activation state of biological sample based on the comparing step.

In some embodiments, the disclosure is directed to a computer-readable storage device comprises instructions to perform::
(a) comparing a biological state of Interleukin 4-Induced gene 1 (IL4I1) from a sample to the biological state of IL4I1 from a control sample; and
(e) determining the AHR activation state of biological sample based on the comparing step.

The present invention thus relates to the following items.

Item 1. A method for detecting a modulation of AHR in a cell or subject, comprising detecting a change of the biological state of IL4I1 in a biological sample derived from said cell or subject, wherein a change in said biological state of IL4I1 in said sample, when compared to a control sample, indicates an IL4I1-related modulation of AHR in said cell or subject.

Item 2. The method according to Item 1, wherein said modulation is selected from an activation or repression of AHR.

Item 3. The method according to Item 1 or 2, wherein said modulation is indicative for an AHR-related physiological or pathological condition in said cell or subject.

Item 4. The method according to Item 3, wherein said physiological or pathological condition is selected from intoxication, cancer, autoimmune disorders, degeneration, inflammation, infection, metabolic diseases and conditions, angiogenesis, drug metabolism, hematopoiesis, lipid metabolism, cell motility, immune modulation, stress conditions, for example, biological, mechanical and environmental stresses.

Item 5. The method according to any one of Items 1 to 4, wherein said biological state as detected is selected from mutations, nucleic acid methylation, copy numbers, expression, amount of protein, protein modifications, cellular localization, metabolites, in particular metabolites as produced by IL4I1, such as, for example, tryptophan degradation products, and a biological activity of IL4I1.

Item 6. The method according to any one of Items 1 to 5, wherein said biological sample is selected from a suitable sample comprising biological fluids, mammalian, for example human, cells, tissues, whole blood, cell lines, cellular supernatants, primary cells, IPSCs, hybridomas, recombinant cells, stem cells, and cancer cells, bone cells, cartilage cells, nerve cells, glial cells, epithelial cells, skin cells, scalp cells, lung cells, mucosal cells, muscle cells, skeletal muscles cells, striated muscle cells, smooth muscle cells, heart cells, secretory cells, adipose cells, blood cells, erythrocytes, basophils, eosinophils, monocytes, lymphocytes, T-cells, B-cells, neutrophils, NK cells, regulatory T-cells, dendritic cells, Th17 cells, Th1 cells, Th2 cells, myeloid cells, macrophages, monocyte derived stromal cells, bone marrow cells, spleen cells, thymus cells, pancreatic cells, oocytes, sperm, kidney cells, fibroblasts, intestinal cells, cells of the female or male reproductive tracts, prostate cells, bladder cells, eye cells, corneal cells, retinal cells, sensory cells, keratinocytes, hepatic cells, brain cells, kidney cells, and colon cells, and the transformed counterparts of said cells or tissues.

Item 7. The method according to any one of Items 1 to 6, wherein said subject is selected from a mammalian subject, for example a human subject, for example a human patient suffering from an AHR-related physiological or pathological condition.

Item 8. The method according to any one of Items 1 to 7, wherein said control sample is selected for example from a sample from a healthy subject or group of subjects.

Item 9. A method for screening for at least one modulator of the biological state of IL4I1, comprising contacting at least one candidate modulator compound with a biological sample, and detecting the modulation of the biological state of IL4I1 or a gene encoding for IL4I1, wherein said modulation or activity identifies a modulator of said biological state.

Item 10. The method according to Item 9, wherein said method further comprises detecting a change of a biological state of IL4I1 in a biological sample, wherein a change in the biological state of said IL4I1 in the presence of said at least one modulator compared to the absence of said at least one modulator identifies a modulator.

Item 11. The method according to Item 9 or 10, wherein said biological state as detected is selected from mutations, nucleic acid methylation, copy numbers, expression, amount of protein, protein modifications, cellular localization, metabolites, in particular metabolites as modulated by IL4I1, such as, for example, tryptophan degradation products, and the biological activity of IL4I1.

Item 12. The method according to any one of Items 9 to 11, wherein said modulator is selected from an inhibitor or an inducer of said biological state of IL4I1.

Item 13. The method according to any one of Items 9 to 12, wherein said method further comprises detecting a modulation of AHR.

Item 14. The method according to any one of Items 9 to 13, wherein said compound is selected from a proteinaceous domain, a small molecule, a peptide, antibodies, for example monoclonal antibodies binding IL4I1, an environmental substance, probiotic, toxin, aerosol, medicine, nutrient, galenic composition, plant extract, volatile compound, homeopathic substance, incense, pharmaceutical drug, vaccine, a compounds or compound mixture derived from organisms, for example animals, plants, fungi, bacteria, archaea, a chemical compound, a compound used in food or cosmetic industry, and a library of said compounds.

Item 15. A method for monitoring the modulation of the biological state of AHR in response to at least one compound, comprising performing a method according to any one of Items 1 to 8 on a biological sample that was contacted with an amount of said at least one compound, and wherein said biological sample is compared to a control sample that was not contacted with said amount of said compound.

Item 16. The method according to Item 15, wherein said biological samples are obtained through the course of a treatment, and/or are compared to a suitable control sample or a sample derived from a group of subjects or patients, as described herein.

Item 17. The method according to any one of Items 1 to 16, wherein said method further comprises the step of using said comparison for unsupervised clustering or supervised classification of said samples into subgroups of IL4I1 modulation, and optionally for further unsupervised clustering or supervised classification of said samples into different subgroups of AHR modulation.

Item 18. The method according to any one of Items 1 to 16, further comprising a stratification of said subject into a particular group of subjects or patient groups.

Item 19. The method according to any one of Items 1 to 18, wherein said method comprises using a high-throughput method.

Item 20. A diagnostic kit comprising materials for performing a method according to any one of Items 1 to 19 in one or separate containers, optionally together with auxiliary agents and/or instructions for performing said method.

Item 21. Use of a diagnostic kit according to Item 20 for a method according to any one of Items 1 to 19.

Item 22. A method for treating and/or preventing an AHR-related disease or condition in a cell, for example in a patient in need of said treatment, comprising performing a method according to any one of Items 1 to 19, and providing a suitable treatment to said patient, wherein said treatment is based, at least in part, on the results of said method.

**Table 1 - Amino acids and metabolites modulated by IL4I1**

| **Chemical Name** | **InChI Key** | **PubChem CID** |
|---|---|---|
| 3-Phenylpyruvic acid | BTNMPGBKDVTSJY-UHFFFAOYSA-N | 997 |
| Indole-3-pyruvic acid | RSTKLPZEZYGQPY-UHFFFAOYSA-N | 803 |
| 4-Hydroxyphenylpyruvic acid | KKADPXVIOXHVKN-UHFFFAOYSA-N | 979 |
| 2-Phenylacetic acid | WLJVXDMOQOGPHL-UHFFFAOYSA-N | 999 |
| 4-Hydroxybenzaldehyde | RGHHSNMVTDWUBI-UHFFFAOYSA-N | 126 |
| 2-Hydroxy-2-phenylacetic acid | IWYDHOAUDWTVEP-UHFFF AOYSA-N | 1292 |
| 3-Indoleacetic acid | SEOVTRFCIGRIMH-UHFFFAOYSA-N | 802 |
| 1H-Indole-3-carboxaldehyde | OLNJUISKUQQNIM-UHFFFAOYSA-N | 10256 |
| Indole-3-lactic acid | XGILAAMKEQUXLS-UHFFFAOYSA-N | 92904 |
| 4-Hydroxyquinoline-2-carboxylic acid | HCZHHEIFKROPDY-UHFFFAOYSA-N | 3845 |
| 1,3-di(1H -indol-3-yl)acetone | OULRFLUQRMGBEN-UHFFFAOYSA-N | 11483104 |
| (3*Z*)-1-(1*H*-indol-3-yl)-3-indol-3-ylidenepropan-2-one | BHYVQPWGWDXGBR-UKTHL TGXSA-N | 57345798 |
| L-valine | KZSNJWFQEVHDMF-BYPYZUCNSA-N | 6287 |
| L-isoleucine | AGPKZVBTJJNPAG-WHFBIAKZSA-N | 6306 |
| L-leucine | ROHFNLRQFUQHCH-YFKPBYRVSA-N | 6106 |
| L-alanine | QNAYBMKLOCPYGJ-REOHCLBHSA-N | 5950 |
| L-glutamic acid | WHUUTDBJXJRKMK-VKHMYHEASA-N | 33032 |
| L-methionine | FFEARJCKVFRZRR-BYPYZUCNSA-N | 6137 |
| L-glutamine | ZDXPYRJPNDTMRX-VKHMYHEASA-N | 5961 |
| 4-methylsulfanyl-2-oxobutanoate | SXFSQZDSUW ACKX-UHFFF AOYSA-M | 4584184 |
| Alpha-Keto-isoleucine | JVQYSWDUAOAHFM-BYPYZUCNSA-N | 439286 |
| alpha-Ketoisovalerate | QHKABHOOEWYVLI-UHFFF AOYSA-N | 49 |
| alpha-Ketoisocaproic acid | BKAJNAXTPSGJCU-UHFFFAOYSA-N | 70 |
| L-proline | ONIBWKKTOPOVIA-BYPYZUCNSA-N | 145742 |
| alpha-ketoglutaric acid | KPGXRSRHYNQIFN-UHFFFAOYSA-N | 51 |

The invention shall now be further described in the following examples with reference to the accompanying figures, nevertheless, without being limited thereto. For the purposes of the present invention, all references as cited herein are incorporated by reference in their entireties.

Figure 1 shows that IL4I1 is expressed in various tumor entities. **a**, heatmap representation of the median log2 transcript per million (log2 TPM) of IDO1, IDO2, TDO2 and IL4I1 expression in the Genotype-Tissue Expression dataset (GTEX) comprising 30 non-diseased tissues. Empty cells denote no expression was detected. Dot size and shading colors correspond to the expression level, light grey denoting low expression and dark grey denoting high expression levels. **b**, heatmap representation of the median log2 transcript per million (log2 TPM) of IDO1, IDO2, TDO2 and IL4I1 expression in 32 TCGA tumors.

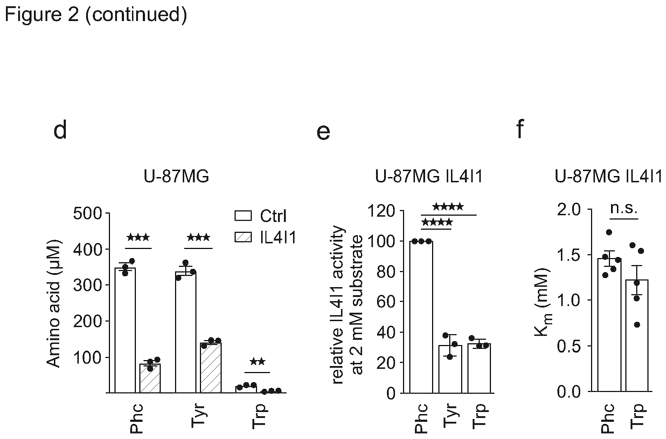

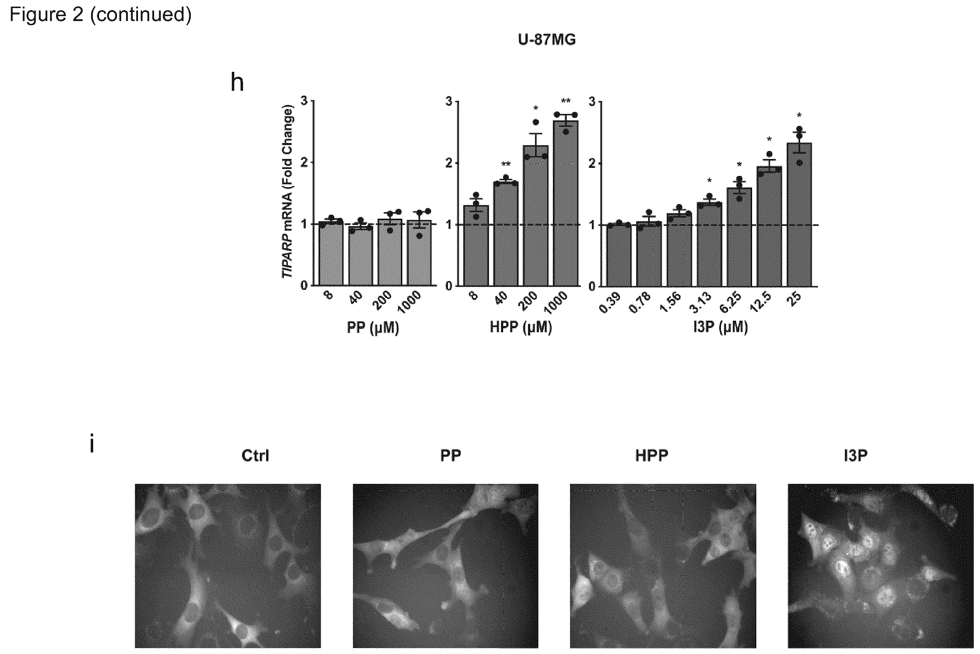

Figure 2 shows that IL4I1 activates the AHR. **a,** mRNA expression of selected AHR target genes in shCtrl and shAHR U-87MG cells expressing IL4I1, relative to shCtrl U-87MG cells without IL4I1 expression (dashed line), cultured for 120 h (*n* = 3 for *EGR1, IL1B, MMP1*; *n* = 4 for all other genes). **b**, Immunoblot (left) and quantification (right) of the nuclear to cytoplasmic ratio of AHR protein expression, in LN-229 cells upon 4 h treatment with supernatants of Ctrl and IL4I1-expressing U-251MG cells cultured for 120 h (*n* = 3). **c**, *TIPARP* mRNA expression in CAS-1 cells treated with siRNA targeting *IL4I1*, relative to cells treated with siCtrl, cultured for 72 h (*n* = 4). **d**, Concentration of Phe, Tyr and Trp in supernatants of Ctrl and IL4I1-expressing U-87MG cells, cultured for 120 h (*n* = 3). **e**, IL4I1 activity in lysates of U-87MG cells expressing IL4I1 in presence of Phe, Tyr or Trp (*n* = 3). **f**, Kₘ values of IL4I1 for Phe and Trp (*n* = 5). **g**, Volcano plots showing the differential regulation of AHR target genes in microarray data of U-87MG cells exposed to 40 µM of PP (left), HPP (middle) or I3P (right) for 24 h compared to vehicle (*n* = 5). Dark grey data points represent selected regulated AHR target genes. Light grey data points represent all other genes. The vertical dotted lines denote a log2FC of +/- 0.58 and the horizontal dotted line is at a p-value cutoff of 0.01. **h**, *TIPARP* mRNA expression in U-87MG treated with PP, HPP, I3P or vehicle (dashed line) for 24 h (*n* = 3). **i**, Representative images of GFP-Ahr expressing tao BpRc1c cells treated with 25 µM PP, HPP, I3P or vehicle for 4 h. *n* values represent independent experiments. Data are presented as mean ± S.E.M and were analyzed by two-tailed paired student's *t*-test (**c, d, e**), two-tailed unpaired student's *t*-test (**f, h**), one-way ANOVA with Tukey's multiple comparisons test (**g**) or repeated measures ANOVA with Dunnett's multiple comparisons test (**j**). **P* < 0.05, ** *P* < 0.01, *** *P* < 0.001, **** *P* < 0.0001. n.s., not significant. * shCtrl compared to IL4I1-shCtrl; # IL4I1-shCtrl compared to IL4I1-shAHR.

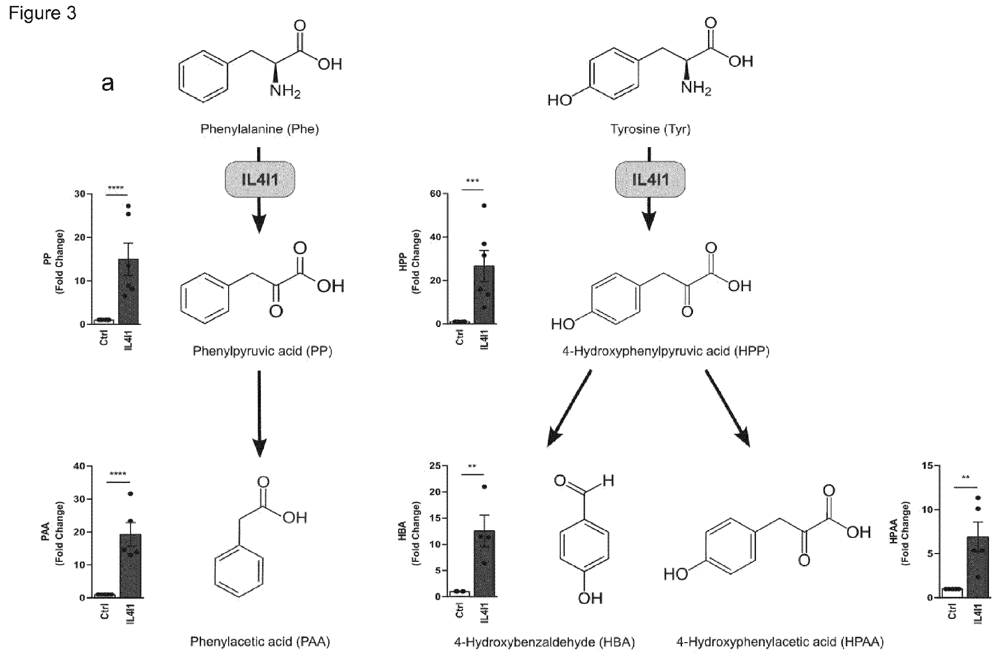

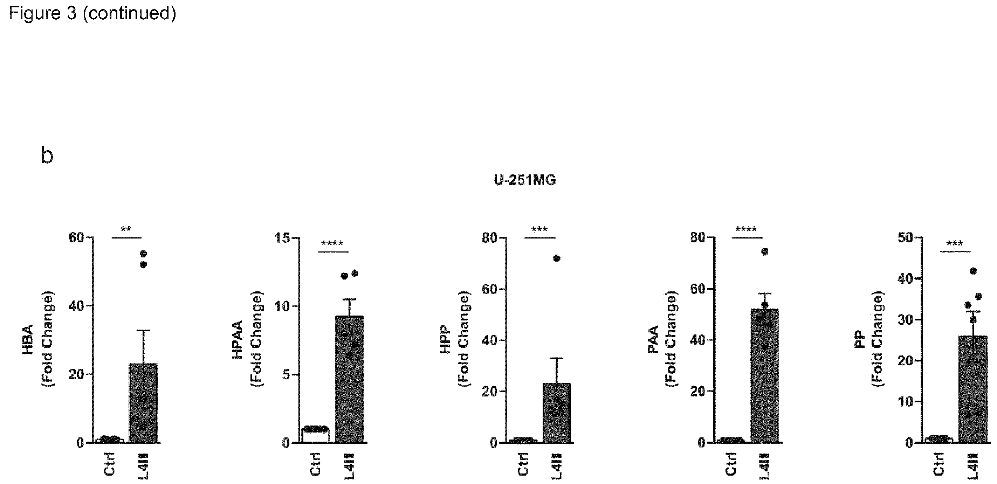

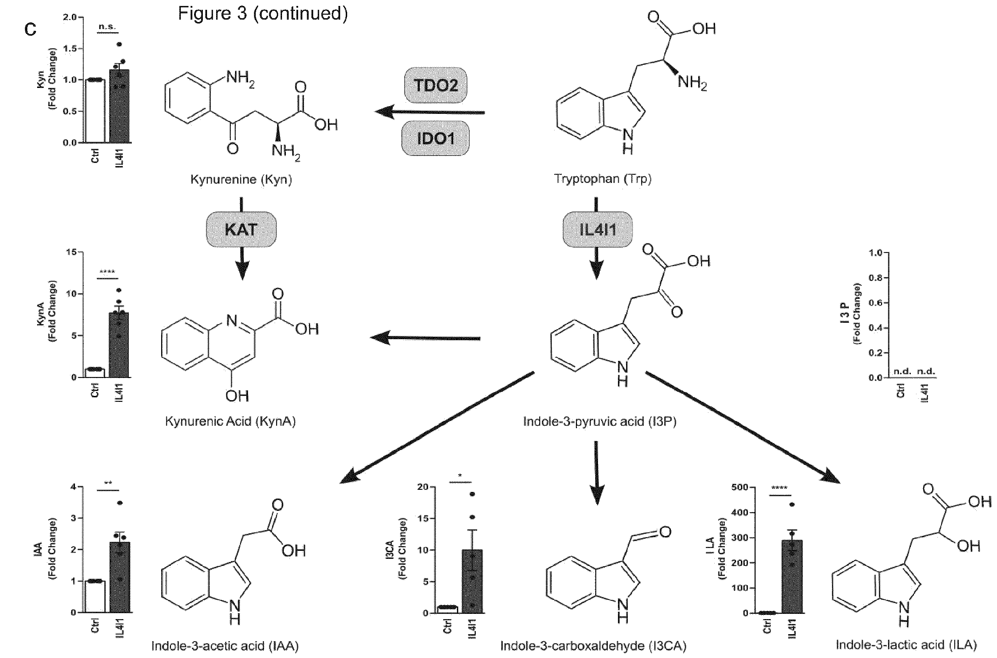

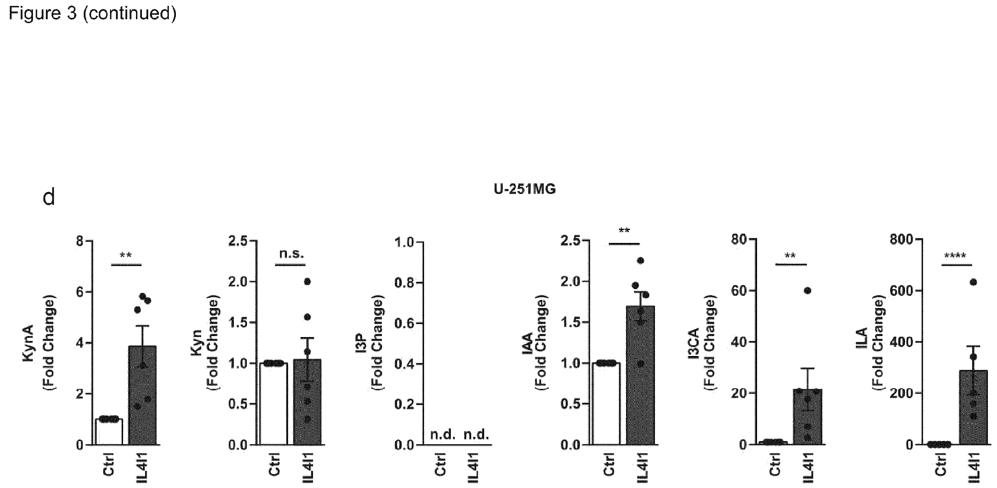

Figure 3 shows that IL4I1 degrades aromatic amino acids and produces AHR ligands. **a**, Phenylpyruvate (PP), phenylacetic acid (PAA), hydroxyphenylpyruvate (HPP), hydroxybenzaldehyde (HBA) and hydroxyphenylacetic acid (HPAA) in the supernatant of U87-ctrl and U87-IL4I1 cells (120 h). **b**, HBA, HPAA, HPP, PAA and PP in the supernatant of U251-ctrl and U251-IL4I1 cells (120 h). **c**, Kynurenine (Kyn), kynurenic acid (KynA), indole-3-pyruvate (I3P), indoleacetic acid (IAA), indole-3-carboxaldehyde (I3CA) and indole-3-lactic acid (ILA) in the supernatant of U87-ctrl and U87-IL4I1 cells (120 h). **d**, KynA, Kyn, I3P, IAA, I3CA and ILA in the supernatant of U251-ctrl and U251-IL4I1 cells (120 h).**P* ≤ 0.05, ****P ≤ 0.01, ****P* ≤ 0.001, *****P* ≤ 0.0001. One sample t test.

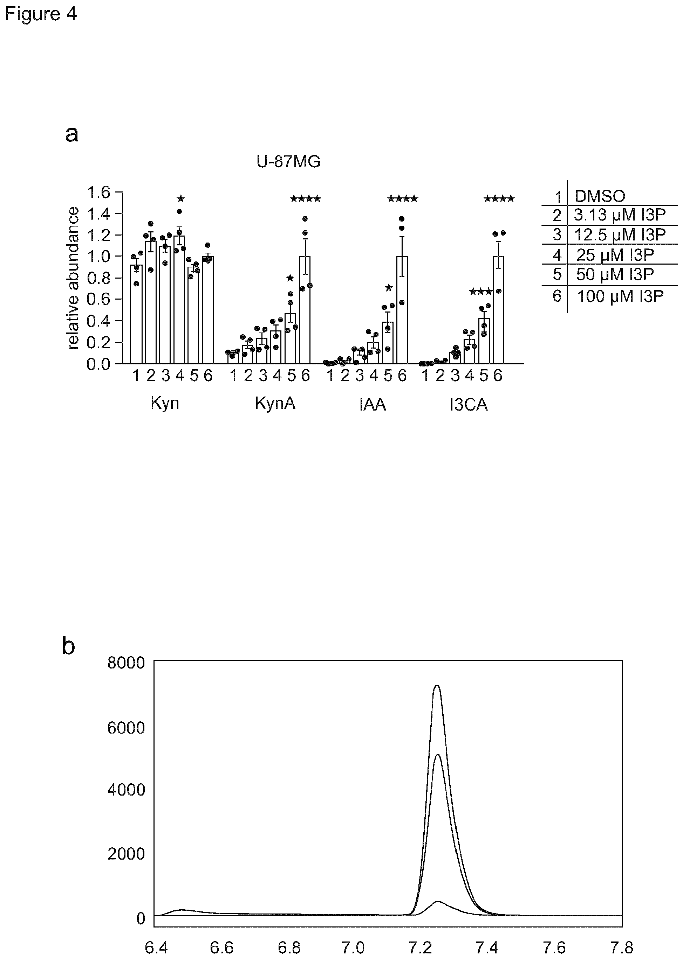

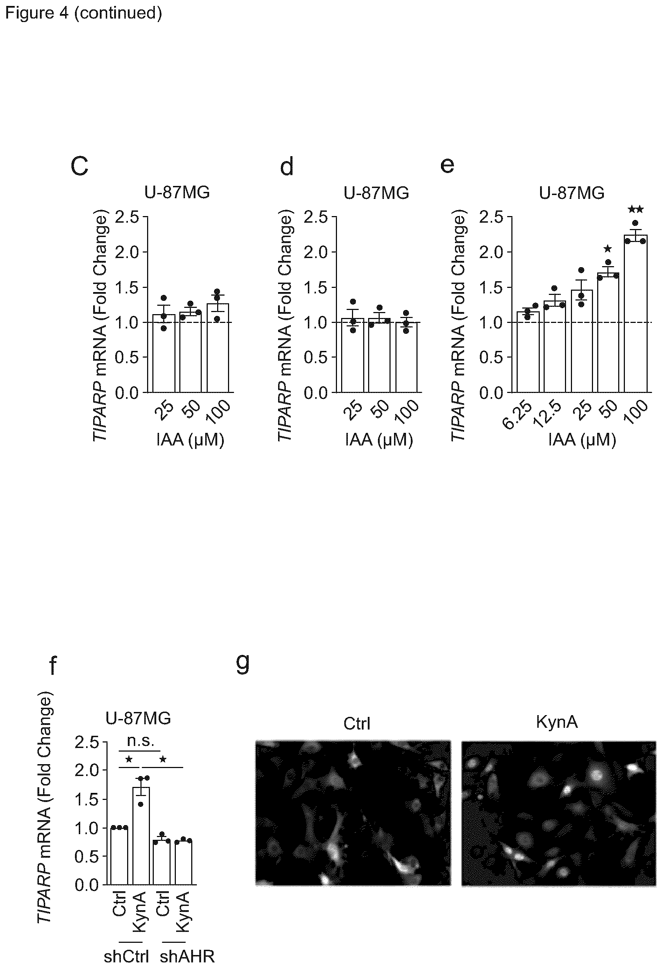

Figure 4 shows the IL4I1-derived Trp metabolites and their effect on AHR activity. **a**, Relative abundance of Kyn, KynA, IAA, and I3CA in supernatants of U-87MG cells treated with I3P or vehicle for 24 h (*n* = 4). **b**, Representative chromatogram showing KynA measured by HPLC with overlay of a KynA standard (black, highest peak, 20 pmol on column). Black, lowest peak: 3.33 mg/mL I3P in phosphate buffered saline (PBS) incubated for 24 h. Grey peak: 3.33 mg/mL I3P incubated in PBS in the presence of 1 mM H₂O₂ for 24 h (n=2). **c-e**, *TIPARP* mRNA expression in U-87MG cells treated with IAA (c), ILA (d), I3CA (e) or vehicle for 24 h (*n* = 3). **f**, *TIPARP* mRNA expression in shCtrl and shAHR U-87MG cells treated with 50 µM KynA or vehicle for 24 h (*n* = 3). **g**, Representative images of GFP-Ahr expressing tao BpRc1c cells treated with either vehicle or 50 µM KynA for 1 h. *n* values represent independent experiments. Data are presented as mean ± S.E.M and were analyzed by two-tailed paired student's *t-*test (a, b, h) or repeated measures ANOVA with Dunnett's multiple comparisons test (c, e-g). **P* < 0.05, ** *P* < 0.01, *** *P* < 0.001, **** *P* < 0.0001. n.s., not significant.

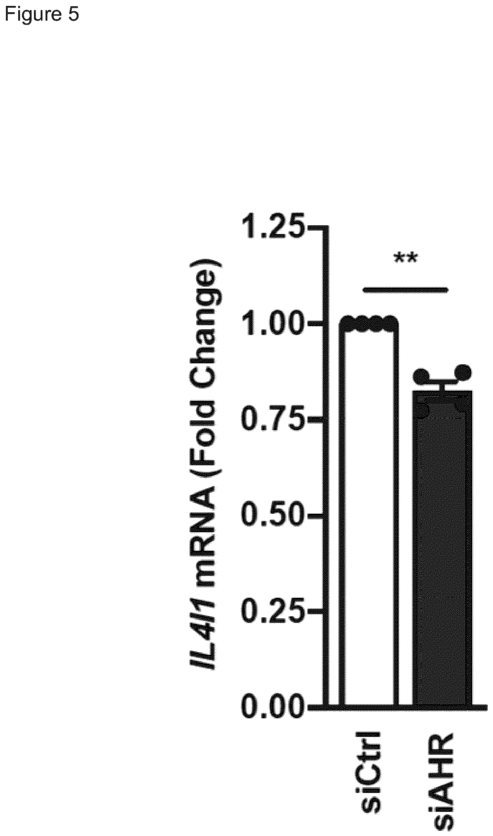

Figure 5 shows that IL4I1 expression is regulated by AHR *IL4I1* mRNA expression in CAS-1 cells treated with siRNA targeting *AHR,* relative to cells treated with siCtrl.

Figure 6 shows a block diagram of the system in accordance with the aspects of the disclosure. CPU: Central Processing Unit ("processor").

Figure 7 shows the flowchart of an exemplary embodiment.

SEQ ID Nos: 1 to 26 show sequences of oligomers as used in the present invention.

### Examples

### Material and Methods

### Microarray and RNA-seq data analysis

Array datasets - The affymetrix microarray chips "human gene 2.0 ST" were analyzed using the oligo package and annotated using NetAffx (14). Raw CEL files were RMA normalized and summarized. Differential gene expression was performed using the *limma* pipeline for microarrays (15).

RNA-seq datasets - The harmonized FPKM data of The Cancer Genome Atlas (TCGA) tumor datasets were downloaded using TCGAbiolinks (16) from GDC (https://gdc.cancer.gov), and only patients with the identifier "primary solid tumor" were retained. The FPKM values were converted to Transcripts per Million (TPMs) (17), TPM data of normal tissues were downloaded from the Genotype-Tissue Expression dataset (GTEX - https://gtexportal.org/home/). All TPM values were log2 transformed.

### Cell culture

HEK293T, LN-229, Tao BpRc1 and U-87MG were obtained from ATCC. CAS-1 and U-251MG were from ICLC and ECACC, respectively. CAS-1, HEK293T, LN-229, U-87MG and U-251MG were cultured in phenol-red free high glucose DMEM medium (Gibco, 31053028) supplemented with 10% FBS (Gibco, 10270106), 2 mM L-glutamine (Gibco, 25030-024), 1 mM sodium pyruvate (Gibco, 11360-039), 100 U/mL penicillin and 100 µg/mL streptomycin (Gibco, 15140-122) (henceforth, referred to as complete DMEM). Tao BpRc1 cells were cultured as above, but with complete phenol-red free DMEM and 5 µg/mL tetracycline (Sigma-Aldrich, T3383). For translocation assays medium with 10% Tet System Approved FBS (Clontech, 631107) was used. Cells were cultured at 37 °C and 5 % CO₂.

### Generation of transgenic cell lines

Human IL4I1 cDNA clone flanked by Gateway compatible recombination sites was purchased from MyBiosource (MBS1270935). The cDNA clone was recombined into Lentivirus compatible Gateway expression vector pLX301 (a gift from D. Root, Addgene plasmid 25895)¹⁸. Production of Lentiviruses was achieved by transfecting HEK293T with pMD2.G (a gift from D. Trono, Addgene plasmid 12259), psPAX2 (a gift from D. Trono, Addgene plasmid 12260), and lentiviral plasmid, using FuGENE HD (Promega, E2311), according to the manufacturer's protocol. Viral supernatants were harvested at 48 h and 72 h, pooled and filtered through a 0.45 µm pore filter. Stable IL4I1 overexpressing (pLX301-IL4I1) and control (pLX301) cell lines were generated by infecting U-87MG and U-251MG cells for 24 hours with respective viral supernatants in presence of 8 µg/mL polybrene (Merck Millipore, TR-1003-G), followed by selection with medium containing 1 µg/mL puromycin (AppliChem, A2856). Stable overexpression of IL4I1 was confirmed by qRT-PCR, western blot and IL4I1 enzymatic activity.

Stable knockdown of AHR in U-87MG cells was achieved using shERWOOD UltramiR Lentiviral shRNA targeting AHR (transOMIC Technologies, TLHSU1400-196-GVO-TRI). Glioma cells were infected with viral supernatants containing either shAHR or shControl (shC) sequences to generate stable cell lines.

shERWOOD UltramiR shRNA sequences are: 
shAHR (ULTRA-3234821): 5'-TGCTGTTGACAGTGAGCGCAGGAAGAATTGTTT
TAGGATATAGTGAAGCCACAGATGTATATCCTAAAACAATTCTTCCTTTGCC
TACTGCCTCGGA-3' (SEQ ID NO: 1);
 
shC (ULTRA-NT#4): 5'- TGCTGTTGACAGTGA GCG
AAGGCAGAAGTATGCAAAGCATTAGTGAAGCCACAGATGTAATGCTTTGCA
TACTTCTGCCTGTGCCTACTGCCTCGG A-3 (SEQ ID NO: 2)'.
 siRNA mediated gene knockdown of *IL4I1* was carried out using ON-TARGETplus Human SMARTpool siRNA reagent (Dharmacon, L-008109-00-0005). siRNA mediated gene knockdown of *AHR* was carried out using ON-TARGETplus Human SMARTpool siRNA reagent (Dharmacon, L-004990-00-0005). siRNA transfections were done with Lipofectamine RNAiMAX (Thermo Fisher Scientific, 13778100), following the manufacturer's protocol. ON-TARGETplus Non-targeting Pool siRNA (Dharmacon, D-001810-10-05) was used as control.

Stably transfected tao BpRc1c cells, expressing a GFP-tagged *Ahr* under tetracycline control, were used to visualize nuclear translocation of Ahr. The murine *Ahr* was cloned into the pEGFP-C1 vector (CLONTECH, Palo Alto, CA) including a tet-off expression system (pRevTRE, CLONTECH). The Phoenix packaging line was used for retroviral transfection into murine hepatoma tao BpRc1 cells deficient of endogenous Ahr expression.

### Cell culture treatment conditions

For treatment of adherent cells with established and hypothetical AHR ligands, 4 x 10⁵ cells per well were seeded in six well plates and incubated for 24 h prior to treatment. Non-adherent cells were seeded in 24 well plates at 5 x 10⁵ cells in 1 mL and treated immediately. For verification of the generated AHR signature, cells were treated with the established AHR agonist Kyn (50 µM, SigmaAldrich, K8625) for 24 h. In order to investigate the potential of direct and downstream IL4I1 metabolites to activate the AHR, cells were treated with I3P (3.125 µM to 100 µM, Sigma-Aldrich, I7017), HPP (8 µM to 1000 µM, Sigma-Aldrich, 114286), PP (8 µM to 1000 µM, Alfa Aesar, L11934), kynurenic acid (50 µM, Sigma-Aldrich, K3375), indole-3-lactic acid (25 µM to 100 µM, Sigma-Aldrich, I5508), 3-indoleacetic acid (25 µM to 100 µM, Sigma-Aldrich, I2886), indole-3-carboxaldehyde (6.25 µM to 100 µM, Sigma-Aldrich, 129445) and supernatants of U-87MG or U-251MG control and IL4I1 expressing cells for 24 h. When using the AHR antagonist SR1 (1µM, Merck Millipore, 182706), cells were treated for 24 h alone or in combination with AHR ligands. DMSO was used for dissolving all compounds such that its final concentration in culture medium did not exceed 0.2 %.

For gene and protein expression experiments involving U-87MG and U-251MG control and IL4I1 expressing cells, 4 x 10⁵ cells per well were seeded in 2 mL in six well plates, and incubated for 72 or 120 h. For metabolomics experiments, cells were seeded at a density of 4 x 10⁵ cells per well in 2 mL of complete DMEM in six well plates and incubated for 24 h. In cases where more cells and supernatant were needed, 2.6 x 10⁶ cells per well were seeded in 13.3 mL of complete DMEM in 10 cm dishes and incubated for 24 h. After 24 h, cells were washed once with PBS, 2 or 13.3 mL of fresh FBS-free DMEM were added (depending on the well size and cell density used) and cells were incubated for 120 h. Sample preparation was adapted from previous studies^{19,20}. Briefly, supernatants were snap frozen in liquid nitrogen and stored at -80°C until metabolite measurement. Plates containing cells were quickly washed once with 37°C pre-heated cell culture grade water, quenched with liquid nitrogen and stored at - 80°C until metabolite measurement.

For experiments where *IL4I1* was knocked down in CAS-1 cells, 4 x 10⁵ cells per well in six well plates were seeded and incubated for 24 h. Cells were transfected with respective control or targeting siRNA. Complete DMEM was replaced with 1.5 mL of FBS-free DMEM 24 h post-transfection and cells were incubated for 72 h.

### RNA isolation and real time PCR

Total RNA was harvested from cultured cells using the RNeasy Mini Kit (Qiagen, 80204) followed by cDNA synthesis using the High Capacity cDNA reverse transcriptase kit (Applied Biosystems, 4368813). A StepOne Plus real-time PCR system (Applied Biosystems) was used to perform real-time PCR of cDNA samples using SYBR Select Master mix (Thermo Scientific, 4309155). Data was processed and analysed using the StepOne Software v 2.3. Relative quantification of target genes was done against *RNA18S* as reference gene using the 2^{ΔΔCt} method. Human primer sequences are listed in table 2 as follows.

**Table 2 Human primer sequences**

| **Gene** | **Forward Primer (5'->3')** | **Reverse Primer (5'->3')** |
|---|---|---|
| *ABCG2* | TTCCACGATATGGATTTACGG (SEQ ID NO: 3) | GTTTCCTGTTGCATTGAGTCC (SEQ ID NO: 4) |
| *AHRR* | CCCTCCTCAGGTGGTGTTTG (SEQ ID NO: 5) | CGACAAATGAAGCAGCGTGT (SEQ ID NO: 6) |
| *CYP1B1* | GACGCCTTTATCCTCTCTGCG (SEQ ID NO: 7) | ACGACCTGATCCAATTCTGCC (SEQ ID NO: 8) |
| *EGRI* | CTGACCGCAGAGTCTTTTCCT (SEQ ID NO: 9) | GAGTGGTTTGGCTGGGGTAA (SEQ ID NO: 10) |
| *EREG* | CTGCCTGGGTTTCCATCTTCT (SEQ ID NO: 11) | GCCATTCATGTCAGAGCTACACT (SEQ ID NO: 12) |
| *ILIB* | CTCGCCAGTGAAATGATGGCT (SEQ ID NO: 13) | GTCGGAGATTCGTAGCTGGAT (SEQ ID NO: 14) |
| *IL4I1* | CGCCCGAAGACATCTACCAG (SEQ ID NO: 15) | GATATTCCAAGAGCGTGTGCC (SEQ ID NO: 16) |
| *MMP1* | GCTAACCTTTGATGCTATAACTACGA (SEQ ID NO: 17) | TTTGTGCGCATGTAGAATCTG (SEQ ID NO: 18) |
| *NPTXI* | CATCAATGACAAGGTGGCCAAG (SEQ ID NO: 19) | GGGCTTGATGGGGTGATAGG (SEQ ID NO: 20) |
| *RNA18S* | GATGGGCGGCGGAAAATAG (SEQ ID NO: 21) | GCGTGGATTCTGCATAATGGT (SEQ ID NO: 22) |
| *SERPINB2* | ACCCCCATGACTCCAGAGAA (SEQ ID NO: 23) | CTTGTG CCTGCAAAATCGCAT (SEQ ID NO: 24) |
| *TIPARP* | CACCCTCTAGCAATGTCAACTC (SEQ ID NO: 25) | CAGACTCGGGATACTCTCTCC (SEQ ID NO: 26) |

### Protein Isolation and Western Blots

For AHR translocation assays, protein content in the nuclear and the cytoplasmic fractions of LN-229 glioma cells was compared by immunoblotting. LN-229 cells were treated with supernatants of U-251MG control or IL4I1 expressing cells (120 h) for 4h. Lysates were snap frozen in liquid nitrogen and thawed three times following 10 cycles of ultrasonication after each freeze-thaw cycle. To isolate protein from the two different cellular fractions NE-PER^{™} Nuclear and Cytoplasmic Extraction Reagents (Thermo Fisher Scientific Inc.) were used. Extraction was performed following the manufacturer's instructions. Nuclear specific Lamin A served as control for appropriate fractionation and was detected using polyclonal rabbit anti-Lamin A (1:500, BioLegend), respectively. AHR was detected using the primary mouse monoclonal anti-AHR antibody clone RPT1 (Abcam, Berlin, Germany).

### AHR nuclear translocation assay

For induction of GFP-Ahr expression in the transgenic tao BpRc1c cells, cells were taken off tetracycline 24 h before translocation assays. Assays were performed in black clear bottom 96-well plates (BD, #353219) with 7500 cells/well in 150 µl/well. Metabolites were added in 50 µL/well induction medium. Cells were exposed for 4h to 25 µM PP, HPP, and I3P respectively. Medium was discarded and cells fixed with prewarmed 3.7% formaldehyde in PBS (10 min at RT). After fixation cells were washed with detergent buffer (DB, 0.01% Tween20 in PBS, Sigma Aldrich), followed by permeabilization with 0.1% Triton X-100 in PBS (Sigma Aldrich) for 15 min at RT. Cells were washed twice with DB and incubated with 0.4 ng/ml Hoechst 33342 (Sigma Aldrich) for 30 min at RT protected from light. After washing with DB and PBS cells were stored in PBS at 4°C until translocation analysis. Translocation by KynA was monitored by live cell imaging after 3 h of exposure including the appropriate negative medium control. Translocation of Ahr by the different metabolites was monitored on a BD Pathway Imager 855.

### Metabolomic analyses

Consumption of phenylalanine, tyrosine and Trp by IL4I1-expressing and non-expressing U-87MG and U-251MG cells was assessed by quantification of the amino acids in the cell culture supernatants after 120 h incubation. For phenylalanine and tyrosine detection, amino acids were labeled with the fluorescent dye AccQ-Tag^{™} (Waters) according to the manufacturer's protocol. The derivatization product was separated at 42 °C on an Acquity BEH C18 column (Waters) using an Acquity H-class UPLC system (Waters) coupled to an Acquity fluorescence detector (FLR) (Waters). Samples were analyzed on the UPLC as described before by Yang et. al., 2015²¹. For analyses of Trp consumption, supernatants were mixed with an equal volume of 12% perchloric acid and incubated on ice for 10 min. Prior to analysis, samples were centrifuged for 10 min at 4 °C and 16,400 g to precipitate proteins and remove remaining cell debris. Metabolites were then separated by reversed phase chromatography on an Acquity HSS T3 column (100 mm x 2.1 mm, 1.7 µm, Waters) connected to an Acquity H-class UPLC system (Waters). The column was heated to 37 °C and equilibrated with 5 column volumes of 100% solvent A (20 mM sodium acetate, 3 mM zinc acetate, pH 6) at a flow rate of 0.55 mL/min. Clear separation of Trp was achieved by increasing the concentration of solvent B (acetonitrile) in solvent A as follows: 4 min 0% B, 10 min 5% B, 13 min 15% B, 15 min 25% B, and return to 0% B in 3 min. Trp was detected by fluorescence (Acquity FLR detector, Waters, excitation: 254 nm, emission: 401 nm). Standards were used for quantification (Sigma). Data acquisition and processing was performed with the Empower3 software suite (Waters).

We took an untargeted metabolomics approach to identify metabolites that were differentially abundant in supernatants of IL4I1- expressing versus non-expressing U-87MG and U-251MG cells cultured for 120 h. To this end, 50 µl of cell culture supernatants were mixed with 200 µl ice-cold acetonitrile by vortexing followed by incubation at -20 °C for 1 h. Samples were centrifuged for 15 min at 4 °C and transferred into TruView UPLC-MS vials (Waters). A pool sample was prepared by mixing equal volumes of all samples. Samples were measured using an I-class UPLC system coupled to a Vion IMS QTof MS (Waters). Metabolites were separated using either a Cogent Diamond Hydride 2.0 column (150x2.1 mm, 2.2 µm; MicroSolv USA) or a HSS T3 column (100x2.1 mm, 1.8 µm; Waters). For instrument control and acquisition of MS data UNIFI 1.8.2 (Waters) was used. Follow-up data analyses were performed using Progenesis QI (Waters). ILA was detected at 204.0662 Da (neg. mode; expected monoisotopic mass: 205.0739 Da; deviation -2 ppm) and identified by the expected fragment ions at 116.0495, 128.0495, 130.0652 and 204.0655 Da. Selected differentially abundant Trp-, phenylalanine- and tyrosine-derived metabolites (IAA, I3CA, KynA, PP, HPP, HBA) and further downstream transformation products (Kyn, PAA, HPAA) were identified by comparing their fragmentation patterns resulting from suspect LC-MS measurements using an HPLC (Agilent 1290) coupled to a triple quad MS (Agilent 6460) with external standards.

For targeted quantification of the metabolites, MRM mode was used. For all test compounds 10 mM stock solutions were prepared by gravimetrically adding the required amount into 1.5 mL Eppendorf safe-lock tubes and dissolving the test compound in 1 mL DMSO. For each compound, stock solutions covered a concentration range from 10 mM to 0.039 mM. For quantification of the metabolites, 300 µL of each bioassay supernatant was added into Eppendorf safe-lock tubes. Associated calibration samples were prepared by adding 300 µL cell culture media and 1 µL of test compound stock solution into Eppendorf safe-lock tubes. Subsequently, 300 µL acetonitrile was added to trigger precipitation of media components. All samples were centrifuged at 8000 rpm for 4 min and 150 µL of the supernatants was transferred into 1.5 mL glass vials equipped with 200 µL glass inserts for HPLC analysis. 5 µl of the sample was injected for the analysis. Water and acetonitrile with 0.1% formic acid were used as eluent A and B, respectively for HPLC separation for 5 min with a flowrate of 0.5 mL/min. Separation was achieved using a 50 mm long Poroshell 120 EC-C18 2.7 micron column (Agilent). The Agilent Jetstream ESI source was set to gas and gas sheath temperature of 300 °C, with a gas flow of 10 L/min and sheath gas flow of 11 L/min. The nebulizer pressure was set to 55 psi and capillary voltage at 2000 V throughout the run. For instrument control and data acquisition MassHunter software suite (Agilent) was used.

### IL4I1 activity assay

U-87MG and U-251MG cells stably expressing IL4I1 and control transduced counterparts were lysed in 0.1% Triton X-100/PBS. Human tissue obtained from resection of metastatic melanoma was lysed in 1% Triton X-100/PBS by shaking with stainless steel beads in a Mixer Mill MM 301 for two cycles of 1 min at 40 Hz. IL4I1 activity was determined by measuring H₂O₂ production via Amplex^{®} Red fluorescence (excitation at 530 nm and emission at 590 nm) every minute for 60 minutes in black 96-well plates using a CLARIOstar^{®} (BMG LABTECH) plate reader. Reactions were prepared in PBS and contained 50 µM Amplex^{®} Red (Cayman Chemicals, #Cay10010469), 0.1 U/mL HRP (Merck Millipore, #516531) and amino acids as IL4I1 substrates as indicated in figure legends. IL4I1 independent H₂O₂ production was assessed in absence of amino acids and subtracted from activity obtained in presence of substrate. IL4I1 mediated H₂O₂ production was calculated using an H₂O₂ calibration curve (0-10 µM final) and normalized to sample protein content as quantified by Bradford assay.

### Software and statistics

Graphical and statistical analysis of gene (real time-PCR) and protein expression data, as well as metabolite data were done using GraphPad Prism software versions 6.0 and 8.0. Unless otherwise indicated, data represents the mean ± S.E.M of at least 3 independent experiments. In cases where data was expressed as fold of change, these values were Log10 transformed and the resulting values were used for statistical analysis. Depending on the data, the following statistical analyses were applied: two-tailed student's t-test (paired or unpaired), one-way ANOVA with Tukey's multiple comparisons test and repeated measures ANOVA with Dunnett's multiple comparisons test. Significant differences were reported as *p < 0.05, ** p < 0.01, *** p < 0.001, **** p < 0.0001. n.s. indicates no significant difference.

When comparing the expression levels of Trp degrading enzymes in normal (GTEX) and tumor tissue, the inventors found that IL4I1 expression was enhanced in cancer tissues compared to normal tissues, similar to IDO1 and TDO2, other tryptophan degrading enzymes that are implicated in activating AHR, (Fig. 1a,b). The inventors show that qRT-PCR of AHR target genes confirmed AHR activation mediated by IL4I1 (Fig. 2a). Further confirming IL4I1-mediated AHR activation, increased nuclear/cytoplasmic localization of the AHR was detected in glioblastoma cells treated with supernatants of the IL4I1-expressing cells (Fig. 2b). Conversely, knockdown of IL4I1 in glioblastoma cells, which constitutively express IL4I1, decreased the expression of the AHR target gene TIPARP (Fig. 2c). Taken together, the inventors' results reveal that IL4I1 indeed activates the AHR.

Next, the inventors set out to investigate how IL4I1 activates the AHR. In keeping with previous reports, IL4I1 expression reduced the levels of phenylalanine, tyrosine and tryptophan (Fig. 2d) with phenylalanine being catabolized most efficiently (Fig. 2e,f). IL4I1 converts phenylalanine, tyrosine and tryptophan to phenylpyruvic acid (PP), hydroxyphenylpyruvic acid (HPP), and indole-3-pyruvic acid (I3P), respectively (1). The inventors therefore exposed AHR-proficient glioblastoma cells to these metabolites to investigate if they activate the AHR. While PP and HPP did not elicit relevant modulation of AHR target genes, gene expression analyses revealed significant regulation of AHR activation signature genes in response to I3P (Fig. 2g,h), which was AHR-dependent. In line, nuclear translocation of the AHR was observed only in response to I3P (Fig. 2i). In summary, the inventors' results indicate that IL4I1 activates the AHR mainly through generation of I3P, which is in agreement with findings from microbiota-derived I3P and I3P generated by D-amino acid oxidase and aspartate aminotransferase (22-25).

The IL4I1-expressing cells showed high levels of PP and HPP as well as their downstream metabolites phenyl acetic acid (PAA), 4-hydroxybenzaldehyde (HBA) and hydroxyphenyl acetic acid (HPAA) (Fig. 3a,b). To the inventors' surprise the inventors were unable to detect I3P (Fig. 3c,d). However, the inventors detected increased levels of compounds derived from I3P including indole acetic acid (IAA), indole-3-carboxaldehyde (I3CA) and indole-3-lactic acid (ILA) (Fig. 3c,d), suggesting that the metabolic flux through I3P is very rapid. Moreover, the levels of kynurenic acid were elevated in the supernatants of the IL4I1 expressing cells (Fig. 3c,d). Treatment of glioblastoma cells with increasing concentrations of I3P resulted in a dose-dependent increase in IAA, I3CA and kynurenic acid in the cell supernatants (Fig. 4a). One reaction through which IL4I1 could enhance kynurenic acid levels is by transamination of kynurenine (produced by IDO1 and/or TDO2) as kynurenine aminotransferase can use I3P, PP or HPP as amino group acceptors (26). However, kynurenine concentrations were not reduced in IL4I1 expressing cells rendering this hypothesis unlikely (Fig. 3c,d). Kynurenic acid formed spontaneously from I3P, which was enhanced in the presence of H₂O₂ (Fig. 4b), suggesting that the H₂O₂ produced by IL4I1 concomitantly with I3P promotes its conversion to kynurenic acid. Indeed, generation of kynurenic acid from I3P that was produced via tryptophan transamination in rat tissues was described previously (27). While the inventors did not observe relevant induction of the AHR target gene TIPARP in response to IAA or I3L (Fig. 4c, d), I3CA (Fig. 4e) and kynurenic acid (Fig. 4f) induced TIPARP transcripts. AHR activation mediated by kynurenic acid was confirmed by nuclear translocation of the AHR (Fig. 4g). In summary, the inventors' data suggest that IL4I1 activates the AHR through downstream products of I3P including kynurenic acid and I3CA, yielding a mixture of AHR activating compounds.

### References as cited

1 Mason, J. M. et al. IL-4-induced gene-1 is a leukocyte L-amino acid oxidase with an unusual acidic pH preference and lysosomal localization. J Immunol 173, 4561-4567 (2004).
2 Chavan, S. S. *et al.* Characterization of the human homolog of the IL-4 induced gene-1 (Fig1). Biochim Biophys Acta 1576, 70-80 (2002).
3 Chu, C. C. & Paul, W. E. Fig1, an interleukin 4-induced mouse B cell gene isolated by cDNA representational difference analysis. Proc Natl Acad Sci U S A 94, 2507-2512 (1997).
4 Boulland, M. L. et al. Human IL4I1 is a secreted L-phenylalanine oxidase expressed by mature dendritic cells that inhibits T-lymphocyte proliferation. Blood 110, 220-227, doi:10.1182/blood-2006-07-036210 (2007).
5 Carbonnelle-Puscian, A. et al. The novel immunosuppressive enzyme IL4I1 is expressed by neoplastic cells of several B-cell lymphomas and by tumor-associated macrophages. Leukemia 23, 952-960, doi:10.1038/leu.2008.380 (2009).
6 Lasoudris, F. et al. IL4I1: an inhibitor of the CD8(+) antitumor T-cell response in vivo. Eur J Immunol 41, 1629-1638, doi:10.1002/eji.201041119 (2011).
7 Santarlasci, V. et al. Rarity of human T helper 17 cells is due to retinoic acid orphan receptor-dependent mechanisms that limit their expansion. Immunity 36, 201-214, doi:10.1016/j.immuni.2011.12.013 (2012).
8 Cousin, C. et al. The immunosuppressive enzyme IL4I1 promotes FoxP3(+) regulatory T lymphocyte differentiation. Eur J Immunol 45, 1772-1782, doi:10.1002/eji.201445000 (2015).
9 Esser, C., Rannug, A. & Stockinger, B. The aryl hydrocarbon receptor in immunity. Trends Immunol 30, 447-454, doi:S1471-4906(09)00131-8 [pii]10.1016/j.it.2009.06.005 (2009).
10 Marshall, N. B. & Kerkvliet, N. I. Dioxin and immune regulation: emerging role of aryl hydrocarbon receptor in the generation of regulatory T cells. Annals of the New York Academy of Sciences 1183, 25-37, doi:10.1111/j.1749-6632.2009.05125.x (2010).
11 Veldhoen, M. et al. The aryl hydrocarbon receptor links TH17-cell-mediated autoimmunity to environmental toxins. Nature 453, 106-109, doi:nature06881 [pii] 10.1038/nature06881 (2008).
12 Platten, M., Nollen, E. A. A., Rohrig, U. F., Fallarino, F. & Opitz, C. A. Tryptophan metabolism as a common therapeutic target in cancer, neurodegeneration and beyond. Nature reviews. Drug discovery, doi:10.1038/s41573-019-0016-5 (2019).
13 Rothhammer, V. & Quintana, F. J. The aryl hydrocarbon receptor: an environmental sensor integrating immune responses in health and disease. Nat Rev Immunol, doi:10.1038/s41577-019-0125-8 (2019).
14 Carvalho, B., Bengtsson, H., Speed, T. P. & Irizarry, R. A. Exploration, normalization, and genotype calls of high-density oligonucleotide SNP array data. Biostatistics 8, 485-499, doi:10.1093/biostatistics/kx1042 (2007).
15 Ritchie, M. E. et al. limma powers differential expression analyses for RNA-sequencing and microarray studies. Nucleic Acids Res 43, e47, doi:10.1093/nar/gkv007 (2015).
16 Colaprico, A. et al. TCGAbiolinks: an R/Bioconductor package for integrative analysis of TCGA data. Nucleic Acids Res 44, e71, doi:10.1093/nar/gkv1507 (2016).
17 Li, B. & Dewey, C. N. RSEM: accurate transcript quantification from RNA-Seq data with or without a reference genome. BMC bioinformatics 12, 323, doi:10.1186/1471-2105-12-323 (2011).
18 Yang, X. et al. A public genome-scale lentiviral expression library of human ORFs. Nat Methods 8, 659-661, doi:10.1038/nmeth.1638 (2011).
19 Lorenz, M. A., Burant, C. F. & Kennedy, R. T. Reducing time and increasing sensitivity in sample preparation for adherent mammalian cell metabolomics. Anal Chem 83, 3406-3414, doi:10.1021/ac103313x (2011).
20 Ivanisevic, J. et al. Toward 'omic scale metabolite profiling: a dual separation-mass spectrometry approach for coverage of lipid and central carbon metabolism. Anal Chem 85, 6876-6884, doi:10.1021/ac401140h (2013).
21 Yang, Y. et al. Relation between chemotaxis and consumption of amino acids in bacteria. Mol Microbiol 96, 1272-1282, doi:10.1111/mmi.13006 (2015).
22 Aoki, R., Aoki-Yoshida, A., Suzuki, C. & Takayama, Y. Indole-3-Pyruvic Acid, an Aryl Hydrocarbon Receptor Activator, Suppresses Experimental Colitis in Mice. J Immunol 201, 3683-3693, doi:10.4049/jimmunol.1701734 (2018).
23 Bittinger, M. A., Nguyen, L. P. & Bradfield, C. A. Aspartate aminotransferase generates proagonists of the aryl hydrocarbon receptor. Mol Pharmacol 64, 550-556, doi:10.1124/mol.64.3.550 (2003).
24 Chowdhury, G. et al. Structural identification of Diindole agonists of the aryl hydrocarbon receptor derived from degradation of indole-3-pyruvic acid. Chem Res Toxicol 22, 1905-1912, doi:10.1021/tx9000418 (2009).
25 Nguyen, L. P. et al. D-amino acid oxidase generates agonists of the aryl hydrocarbon receptor from D-tryptophan. Chem Res Toxicol 22, 1897-1904, doi:10.1021/tx900043s (2009).
26 Han, Q., Li, J. & Li, J. pH dependence, substrate specificity and inhibition of human kynurenine aminotransferase I. Eur J Biochem 271, 4804-4814, doi:10.1111/j.1432-1033.2004.04446.x (2004).
27 Russi, P., Carla, V. & Moroni, F. Indolpyruvic acid administration increases the brain content of kynurenic acid. Is this a new avenue to modulate excitatory amino acid receptors in vivo? Biochem Pharmacol 38, 2405-2409 (1989).

## Claims

1. A method for detecting a modulation of AHR in a cell or subject, comprising detecting a change of the biological state of IL4I1 in a biological sample derived from said cell or subject, wherein a change in said biological state of IL4I1 in said sample, when compared to a healthy control sample or an internal standard, indicates an IL4I1-related modulation of AHR in said cell or subject, wherein preferably said modulation is selected from an activation or repression of AHR.

2. The method according to claim 1, wherein said modulation is indicative for an AHR-related physiological or pathological condition in said cell or subject, preferably wherein said physiological or pathological condition is selected from intoxication, cancer, autoimmune disorders, degeneration, inflammation, infection, metabolic diseases and conditions, angiogenesis, drug metabolism, hematopoiesis, lipid metabolism, cell motility, immune modulation, stress conditions, for example, biological, mechanical and environmental stresses.

3. The method according to claim 1 or 2, wherein said biological state as detected is selected from mutations, nucleic acid methylation, copy numbers, expression, amount of protein, protein modifications, cellular localization, metabolites, in particular metabolites as produced by IL4I1, such as, for example, tryptophan degradation products, and a biological activity of IL4I1.

4. The method according to any one of claims 1 to 3, wherein said biological sample is selected from a suitable sample comprising biological fluids, mammalian, for example human, cells, tissues, whole blood, cell lines, cellular supernatants, primary cells, IPSCs, hybridomas, recombinant cells, stem cells, and cancer cells, bone cells, cartilage cells, nerve cells, glial cells, epithelial cells, skin cells, scalp cells, lung cells, mucosal cells, muscle cells, skeletal muscles cells, striated muscle cells, smooth muscle cells, heart cells, secretory cells, adipose cells, blood cells, erythrocytes, basophils, eosinophils, monocytes, lymphocytes, T-cells, B-cells, neutrophils, NK cells, regulatory T-cells, dendritic cells, Th17 cells, Th1 cells, Th2 cells, myeloid cells, macrophages, monocyte derived stromal cells, bone marrow cells, spleen cells, thymus cells, pancreatic cells, oocytes, sperm, kidney cells, fibroblasts, intestinal cells, cells of the female or male reproductive tracts, prostate cells, bladder cells, eye cells, corneal cells, retinal cells, sensory cells, keratinocytes, hepatic cells, brain cells, kidney cells, and colon cells, and the transformed counterparts of said cells or tissues.

5. The method according to any one of claims 1 to 4, wherein said subject is selected from a mammalian subject, for example a human subject, for example a human patient suffering from an AHR-related physiological or pathological condition.

6. The method according to any one of claims 1 to 5, wherein said biological state of IL4I1 in said sample is compared to said healthy control sample, which is selected from a sample from a healthy subject or group of subjects.

7. A method for screening for at least one modulator of the biological state of IL4I1, comprising contacting at least one candidate modulator compound with a biological sample, and detecting the modulation of the biological state of IL4I1 or a gene encoding for IL4I1, wherein said modulation or activity identifies a modulator of said biological state, wherein said method preferably further comprises detecting a change of a biological state of IL4I1 in a biological sample, wherein a change in the biological state of said IL4I1 in the presence of said at least one modulator compared to the absence of said at least one modulator identifies a modulator, and wherein said method further comprises detecting a modulation of AHR.

8. The method according to claim 7, wherein said biological state as detected is selected from mutations, nucleic acid methylation, copy numbers, expression, amount of protein, protein modifications, cellular localization, metabolites, in particular metabolites as modulated by IL4I1, such as, for example, tryptophan degradation products, and the biological activity of IL4I1.

9. The method according to any one of claims 7 or 8, wherein said modulator is selected from an inhibitor or an inducer of said biological state of IL4I1.

10. The method according to any one of claims 7 to 9, wherein said compound is selected from a proteinaceous domain, a small molecule, a peptide, antibodies, for example monoclonal antibodies binding IL4I1, an environmental substance, probiotic, toxin, aerosol, medicine, nutrient, galenic composition, plant extract, volatile compound, homeopathic substance, incense, pharmaceutical drug, vaccine, a compounds or compound mixture derived from organisms, for example animals, plants, fungi, bacteria, archaea, a chemical compound, a compound used in food or cosmetic industry, and a library of said compounds.

11. A method for monitoring the modulation of the biological state of AHR in response to at least one compound, comprising performing a method according to any one of claims 1 to 6 on a biological sample that was contacted with an amount of said at least one compound, and wherein said biological sample is compared to a control sample that was not contacted with said amount of said compound, wherein preferably said biological samples are obtained through the course of a treatment, and/or are compared to a suitable control sample or a sample derived from a group of subjects or patients, as described herein.

12. The method according to any one of claims 1 to 11, wherein said method further comprises the step of using said comparison for unsupervised clustering or supervised classification of said samples into subgroups of IL4I1 modulation, and optionally for further unsupervised clustering or supervised classification of said samples into different subgroups of AHR modulation, and optionally further comprising a stratification of said subject into a particular group of subjects or patient groups.

13. A diagnostic kit comprising materials for performing a method according to any one of claims 1 to 12 in one or separate containers, optionally together with auxiliary agents and/or instructions for performing said method.

14. Use of a diagnostic kit according to claim 13 for a method according to any one of claims 1 to 12.

## Patentansprüche

1. Verfahren zum Nachweisen einer Modulation von AHR in einer Zelle oder einem Subjekt, umfassend das Nachweisen einer Änderung des biologischen Zustands von IL411 in einer biologischen Probe, die von der Zelle oder dem Subjekt stammt, wobei eine Änderung des biologischen Zustands von IL411 in der Probe im Vergleich zu einer gesunden Kontrollprobe oder einem internen Standard eine IL4I1-bezogene Modulation von AHR in der Zelle oder dem Subjekt anzeigt, wobei die Modulation vorzugsweise aus einer Aktivierung oder Repression von AHR ausgewählt ist.

2. Verfahren nach Anspruch 1, wobei die Modulation auf einen AHR-bezogenen physiologischen oder pathologischen Zustand in der Zelle oder dem Subjekt hinweist, wobei der physiologische oder pathologische Zustand vorzugsweise aus Intoxikation, Krebs, Autoimmunerkrankungen, Degeneration, Entzündung, Infektion, Stoffwechselkrankheiten und -zustände, Angiogenese, Arzneimittel-metabolismus, Hämatopoese, Lipidmetabolismus, Zellmotilität, Immunmodulation, Belastungszuständen, zum Beispiel biologischen, mechanischen und umweltbedingten Belastungen ausgewählt ist.

3. Verfahren nach Anspruch 1 oder 2, wobei der nachgewiesene biologische Zustand aus Mutationen, Nukleinsäuremethylierung, Kopienzahlen, Expression, Proteinmenge, Proteinmodifikationen, zellulärer Lokalisierung, Metaboliten, insbesondere von IL411 produzierten Metaboliten wie z. B. Tryptophan-Abbauprodukte, und einer biologischen Aktivität von IL411 ausgewählt ist.

4. Verfahren nach einem der Ansprüche 1 bis 3, wobei die biologische Probe aus einer geeigneten Probe ausgewählt ist, umfassend biologische Flüssigkeiten, Säugetier-, beispielsweise menschliche, Zellen, Gewebe, Vollblut, Zelllinien, Zellüberstände, Primärzellen, IPSCs, Hybridome, rekombinante Zellen, Stammzellen, und Krebszellen, Knochenzellen, Knorpelzellen, Nervenzellen, Gliazellen, Epithelzellen, Hautzellen, Kopfhautzellen, Lungenzellen, Schleimhautzellen, Muskelzellen, Skelettmuskelzellen, Zellen von quergestreifter Muskulatur, Zellen von glatter Muskulatur, Herzzellen, sekretorische Zellen, Fettzellen, Blutzellen, Erythrozyten, Basophile, Eosinophile, Monozyten, Lymphozyten, T-Zellen, B-Zellen, Neutrophile, NK-Zellen, regulatorische T-Zellen, dendritische Zellen, Th17-Zellen, Th1-Zellen, Th2-Zellen, myeloische Zellen, Makrophagen, Stromazellen aus Monozyten, Knochenmarkzellen, Milzzellen, Thymuszellen, Pankreaszellen, Eizellen, Spermien, Nierenzellen, Fibroblasten, Darmzellen, Zellen des weiblichen oder männlichen Fortpflanzungstrakts, Prostatazellen, Blasenzellen, Augenzellen, Hornhautzellen, Netzhautzellen, Sinneszellen, Keratinozyten, Leberzellen, Gehirnzellen, Nierenzellen und Dickdarmzellen sowie die transformierten Gegenstücke besagter Zellen oder Gewebe.

5. Verfahren nach einem der Ansprüche 1 bis 4, wobei das Subjekt aus einem Säugetier-Subjekt, zum Beispiel einem menschlichen Subjekt, zum Beispiel einem menschlichen Patienten, der an einem AHR-bezogenen physiologischen oder pathologischen Zustand leidet, ausgewählt ist.

6. Verfahren nach einem der Ansprüche 1 bis 5, wobei der biologische Zustand von IL411 in der Probe mit der gesunden Kontrollprobe verglichen wird, die aus einer Probe von einem gesunden Subjekt oder einer Gruppe von gesunden Subjekten ausgewählt ist.

7. Verfahren zum Screenen auf mindestens einen Modulator des biologischen Zustands von IL4I1, umfassend das Inkontaktbringen von mindestens einer Modulator-Kandidatenverbindung mit einer biologischen Probe und das Nachweisen der Modulation des biologischen Zustands von IL411 oder eines IL411 codierenden Gens, wobei die Modulation oder Aktivität einen Modulator des biologischen Zustands identifiziert, wobei das Verfahren vorzugsweise ferner das Nachweisen einer Änderung eines biologischen Zustands von IL411 in einer biologischen Probe umfasst, wobei eine Änderung des biologischen Zustands von IL411 in Gegenwart des mindestens einen Modulators im Vergleich zur Abwesenheit des mindestens einen Modulators einen Modulator identifiziert, und wobei das Verfahren ferner das Nachweisen einer Modulation von AHR umfasst.

8. Verfahren nach Anspruch 7, wobei der nachgewiesene biologische Zustand aus Mutationen, Nukleinsäuremethylierung, Kopienzahlen, Expression, Proteinmenge, Proteinmodifikationen, zellulärer Lokalisierung, Metaboliten, insbesondere von IL411 modulierte Metaboliten wie z. B. Tryptophan-Abbauprodukte, und der biologischen Aktivität von IL411 ausgewählt ist.

9. Verfahren nach Anspruch 7 oder 8, wobei der Modulator aus einem Inhibitor oder einem Induktor des biologischen Zustands von IL411 ausgewählt ist.

10. Verfahren nach einem der Ansprüche 7 bis 9, wobei die Verbindung aus einer proteinhaltigen Domäne, einem kleinen Molekül, einem Peptid, Antikörpern, zum Beispiel monoklonalen IL411 bindenden Antikörpern, einer Umweltsubstanz, einem Probiotikum, Toxin, Aerosol, Medikament, Nährstoff, einer galenischen Zusammensetzung, einem Pflanzenextrakt, einer flüchtigen Verbindung, homöopathischen Substanz, Räucherwerk, einem pharmazeutischen Medikament, einem Impfstoff, einer von Organismen stammenden Verbindung oder Mischung von Verbindungen, zum Beispiel von Tieren, Pflanzen, Pilzen, Bakterien, Archaeen, einer chemischen Verbindung, einer in der Lebensmittel- oder Kosmetikindustrie verwendeten Verbindung, und aus einer Bibliothek der besagten Verbindungen ausgewählt ist.

11. Verfahren zum Überwachen der Modulation des biologischen Zustands von AHR als Reaktion auf mindestens eine Verbindung, umfassend die Durchführung eines Verfahrens nach einem der Ansprüche 1 bis 6 an einer biologischen Probe, die mit einer Menge der mindestens einen Verbindung in Kontakt gebracht wurde, und wobei die biologische Probe mit einer Kontrollprobe verglichen wird, die nicht mit der besagten Menge der besagten Verbindung in Kontakt gebracht wurde, wobei die biologischen Proben vorzugsweise im Verlauf einer Behandlung erhalten werden und/oder mit einer geeigneten Kontrollprobe oder einer Probe verglichen werden, die von einer Gruppe von Subjekten oder Patienten stammt, wie hier beschrieben.

12. Verfahren nach einem der Ansprüche 1 bis 11, wobei das Verfahren ferner den Schritt des Verwendens des Vergleichs für unüberwachtes Clustering oder überwachte Klassifizierung der Proben in Untergruppen der IL4I1-Modulation und optional für weiteres unüberwachtes Clustering oder überwachte Klassifizierung der Proben in verschiedene Untergruppen der AHR-Modulation umfasst, und optional ferner eine Stratifizierung des Subjekts in eine bestimmte Gruppe von Subjekten oder Patientengruppen umfasst.

13. Diagnostik-Kit, umfassend Materialien zum Durchführen eines Verfahrens nach einem der Ansprüche 1 bis 12 in einem oder getrennten Behältern, optional zusammen mit Hilfsstoffen und/oder Anweisungen zum Durchführen des Verfahrens.

14. Verwendung eines Diagnostik-Kits nach Anspruch 13 für ein Verfahren nach einem der Ansprüche 1 bis 12.

## Revendications

1. Procédé de détection d'une modulation de l'AHR dans une cellule ou un sujet, comprenant la détection d'un changement de l'état biologique de l'IL411 dans un échantillon biologique dérivé de ladite cellule ou dudit sujet, un changement de l'état biologique de l'IL411 dans ledit échantillon, comparé à un échantillon de contrôle sain ou à un étalon interne, indiquant une modulation de l'AHR due à la présence de l'IL411 dans ladite cellule ou ledit sujet, ladite modulation étant choisie de préférence parmi l'activation ou la répression de l'AHR.

2. Procédé selon la revendication 1, ladite modulation indiquant un état physiologique ou pathologique lié à l'AHR dans ladite cellule ou ledit sujet, ledit état physiologique ou pathologique étant choisi de préférence parmi l'intoxication, le cancer, les maladies auto-immunes, la dégénérescence, l'inflammation, l'infection, les maladies et affections métaboliques, l'angiogenèse, le métabolisme des médicaments, l'hématopoïèse, le métabolisme des lipides, la motilité cellulaire, la modulation immunitaire, les états de stress, par exemple, le stress biologique, le stress mécanique et le stress environnemental.

3. Procédé selon la revendication 1 ou 2, l'état biologique détecté étant choisi parmi les mutations, la méthylation des acides nucléiques, le nombre de copies, l'expression, la quantité de protéines, les modifications des protéines, la localisation cellulaire, les métabolites, en particulier les métabolites produits par l'IL411 tels que les produits de dégradation du tryptophane, par exemple, et une activité biologique de l'IL411.

4. Procédé selon l'une des revendications 1 à 3, ledit échantillon biologique étant tiré d'un échantillon approprié comprenant des fluides biologiques, mammifères, par exemple, humaines, des cellules, des tissus, du sang entier, des lignées cellulaires, des surnageants cellulaires, des cellules primaires, des IPSC, des hybridomes, des cellules recombinantes, des cellules souches, et des cellules cancéreuses, des cellules osseuses, des cellules cartilagineuses, des cellules nerveuses, des cellules gliales, des cellules épithéliales, des cellules cutanées, des cellules du cuir chevelu, des cellules pulmonaires, des cellules muqueuses, des cellules musculaires, des cellules des muscles squelettiques, des cellules des muscles striés, des cellules des muscles lisses, des cellules cardiaques, des cellules sécrétoires, des cellules adipeuses, des cellules sanguines, des érythrocytes, des basophiles, des éosinophiles, des monocytes, des lymphocytes, des cellules T, des cellules B, des neutrophiles, des cellules NK, des cellules T régulatrices, des cellules dendritiques, des cellules Th17, des cellules Th1, des cellules Th2, des cellules myéloïdes, des macrophages, des cellules stromales dérivées de monocytes, des cellules de la moelle osseuse, des cellules de la rate, des cellules du thymus, des cellules du pancréas, des ovocytes, des spermatozoïdes, des cellules rénales, des fibroblastes, des cellules intestinales, des cellules de l'appareil reproducteur féminin ou masculin, des cellules de la prostate, des cellules de la vessie, des cellules oculaires, des cellules de la cornée, des cellules rétiniennes, des cellules sensorielles, des kératinocytes, des cellules hépatiques, des cellules cérébrales, des cellules rénales et des cellules du côlon, ainsi que les équivalents transformés de ces cellules ou tissus.

5. Procédé selon l'une des revendications 1 à 4, ledit sujet étant choisi parmi les mammifères, par exemple, un sujet humain, notamment un patient humain souffrant d'un état physiologique ou pathologique lié à l'AHR.

6. Procédé selon l'une des revendications 1 à 5, l'état biologique de l'IL411 dans ledit échantillon étant comparé à l'échantillon de contrôle sain qui est tiré d'un échantillon provenant d'un sujet sain ou d'un groupe de sujets sains.

7. Procédé de dépistage d'au moins un modulateur de l'état biologique de l'IL411, comprenant la mise en contact d'au moins un composé modulateur candidat avec un échantillon biologique et la détection de la modulation de l'état biologique de l'IL411 ou d'un gène codant pour IL411, ladite modulation ou activité identifiant un modulateur dudit état biologique, ledit procédé comprenant de préférence la détection d'un changement de l'état biologique de l'IL411 dans un échantillon biologique, un changement de l'état biologique de l'IL411 en présence dudit au moins un modulateur par rapport à l'absence dudit au moins un modulateur identifiant un modulateur, et ledit procédé comprenant en outre la détection d'une modulation de l'AHR.

8. Procédé selon la revendication 7, ledit état biologique détecté étant choisi parmi les mutations, la méthylation des acides nucléiques, le nombre de copies, l'expression, la quantité de protéine, les modifications de protéine, la localisation cellulaire, les métabolites, en particulier les métabolites modulés par l'IL411 tels que les produits de dégradation du tryptophane, par exemple, et l'activité biologique de l'IL411.

9. Procédé selon l'une des revendications 7 ou 8, ledit modulateur étant choisi parmi un inhibiteur ou un inducteur de l'état biologique de l'IL411.

10. Procédé selon l'une des revendications 7 à 9, ledit composé étant choisi parmi un domaine protéique, une petite molécule, un peptide, des anticorps, par exemple, des anticorps monoclonaux liant l'IL411, une substance environnementale, un probiotique, une toxine, un aérosol, un médicament, un nutriment, une composition galénique, un extrait végétal, un composé volatil, une substance homéopathique, un encens, un médicament pharmaceutique, un vaccin, un composé ou un mélange de composés dérivés d'organismes, par exemple, d'animaux, de plantes, de champignons, de bactéries, d'archées, un composé chimique, un composé utilisé dans l'industrie alimentaire ou cosmétique, et une banque de ces composés.

11. Procédé de surveillance de la modulation de l'état biologique de l'AHR en réponse à au moins un composé, comprenant la mise en œuvre d'un procédé selon l'une des revendications 1 à 6 sur un échantillon biologique qui a été mis en contact avec une quantité dudit au moins un composé, et ledit échantillon biologique étant comparé à un échantillon de contrôle qui n'a pas été mis en contact avec ladite quantité dudit composé, lesdits échantillons biologiques étant obtenus de préférence au cours d'un traitement et/ou comparés à un échantillon de contrôle approprié ou à un échantillon dérivé d'un groupe de sujets ou de patients, comme décrit ci-dessus.

12. Procédé selon l'une des revendications 1 à 11, ledit procédé comprenant en outre l'étape consistant à utiliser ladite comparaison pour un regroupement non supervisé ou une classification supervisée desdits échantillons en sous-groupes de modulation de l'IL411, et éventuellement pour un regroupement non supervisé supplémentaire ou une classification supervisée desdits échantillons en différents sous-groupes de modulation de l'AHR, et comprenant éventuellement en outre une stratification dudit sujet en un groupe particulier de sujets ou de groupes de patients.

13. Kit de diagnostic comprenant des matériaux pour la mise en œuvre d'un procédé selon l'une des revendications 1 à 12 dans un ou plusieurs récipients séparés, éventuellement accompagnés d'agents auxiliaires et/ou d'instructions pour la mise en œuvre dudit procédé.

14. Utilisation d'un kit de diagnostic selon la revendication 13 pour un procédé selon l'une des revendications 1 à 12.
